# EUROPEAN PATENT APPLICATION

(11) **EP 4 303 304 A1**
(43) Date of publication of application: **10.01.2024**
(21) Application number: 22763145.4
(22) Date of filing: 25.02.2022
(51) Int. Cl.: C12N 15/12, A61K 9/08, A61K 31/7105, A61K 31/713, A61K 35/76, A61K 35/761, A61K 48/00, A61P 25/00, A61P 27/16, A61P 43/00, C12N 5/10, C12N 15/11, C12N 15/113, C12N 15/86, C12Q 1/06

(54) **POLYNUCLEOTIDE FOR TREATMENT OF NEURODEGENERATIVE DISEASE, VECTOR, CELL, PHARMACEUTICAL COMPOSITION, AND SCREENING METHOD**

(30) Priority: 03.03.2021 JP 2021033464
(71) Applicant: RIKEN, Wako-shi, Saitama 351-0198 (JP)
(72) Inventor: KAGEYAMA, Ryoichiro, Wako-shi, Saitama 351-0198 (JP); KAISE, Takashi, Wako-shi, Saitama 351-0198 (JP); FUKUI, Masahiro, Wako-shi, Saitama 351-0198 (JP)
(74) Representative: Mewburn Ellis LLP
(86) International application number: PCT/JP2022/008055
(87) International publication number: WO 2022/186089

(57) **Abstract**

The purpose of the present invention is to provide a novel method for increasing the number of newly generated neurons in the adult brain, and to provide a polynucleotide, a vector, and a pharmaceutical composition for use in the method. The present invention provides: a polynucleotide characterized by including (A) the nucleic acid sequence of the Plagl2 gene, (B) an miR-shRNA (microRNA adapted short hairpin RNA) nucleic acid sequence for the Dyrk1a gene, and (C) a promoter sequence operatively connected to the nucleic acid sequences; a vector including the polynucleotide; and a pharmaceutical composition including the polynucleotide and the vector.

## Description

### TECHNICAL FIELD

The present invention relates to a polynucleotide, a vector, cells, a pharmaceutical composition, and a screening method for treating a neurodegenerative disease.

### BACKGROUND ART

It has become clear that neural stem cells and neural progenitor cells are also present in the adult brain of mammals including a human, although the absolute number thereof is small, and that these cells provide neurogenesis in some brain regions. However, most of the neural stem cells in the adult brain are quiescent and have a low proliferative ability and a neuronproducing ability. These proliferative ability and neuron producing ability further decline with age, and thus there are almost no newly born neurons (newborn neurons) in the aging brain. A remarkable decrease in newborn neurons is also thought to be a cause of brain hypofunction such as dementia, and a neurodegenerative disease such as Alzheimer's disease and Parkinson's disease.

Under such circumstances, an attempt has been made to promote functional recovery by amplifying and differentiating neural stem cells and neural progenitor cells, which are present in small amounts in the adult brain, by stimulation with a growth factor and the like (see Non Patent Documents 1). However, the effect thereof cannot be deemed to be sufficient. In addition, research on a cell transplantation therapy model using neural stem cells and neural progenitor cells derived from a fetal brain, ES cells, and iPS cells is also being actively carried out, but it is not clear even whether or not cell transplantation therapy in the brain can reconstruct a normal neural network, and the risk of a side effect thereof is also high. Further, many attempts have been made so far to increase the number of newborn neurons in the adult brain, but the effects thereof are still limited (see Non Patent Literatures 1 to 5).

### PRIOR ART DOCUMENTS

### Non Patent Documents

Non Patent Document 1: Nagahara AH et al. (2009) Neuroprotective effects of brainderived neurotrophic factor in rodent and primate models of Alzheimer's disease, Nat. Med., vol.15(3), pp.331-337.
Non Patent Document 2: Henriette van Praag et al., Exercise enhances learning and hippocampal neurogenesis in aged mice, J Neurosci., 2005 Sep 21;25(38):8680-5
Non Patent Document 3: Benedetta Artegiani et al., Overexpression of cdk4 and cyclinD1 triggers greater expansion of neural stem cells in the adult mouse brain. J Exp Med. 2011 May 9;208(5):937-48
Non Patent Document 4: Benraiss, A.et al.(2013) Mobilization of endogenous progenitor cells regenerates functionally-integrated medium spiny striopallidal projection neurons and delays disease progression in a transgenic model of Huntington's disease. Cell Stem Cell 12, 787-799
Non Patent Document 5: Berdugo-Vega, G.et al.(2020). Increasing neurogenesis refines hippocampal activity rejuvenating navigational learning strategies and contextual memory throughout life. Nat. Commun. 11, 135

### SUMMARY OF INVENTION

### Technical Problem

An object of the present invention is to provide a novel method for increasing the number of newborn neurons in an adult brain, and also provide a polynucleotide, a vector, transformed cells, and a pharmaceutical composition used in this method, and a method for screening for a substance that increases the number of newborn neurons in an adult brain.

### Solution to Problem

Under these circumstances, the present inventors have carried out intensive studies, and as a result found that by introducing a specific gene set into the aged mouse brain with a lentivirus, endogenous neural stem cells are efficiently activated and proliferated to produce a large number of neurons. Aged neural stem cells reverted to their adolescent or younger state to produce a large number of neurons, resulting in improved memory and learning abilities. In addition, even when the specific gene set was introduced into the brain of an Alzheimer's disease model mouse, endogenous neural stem cells were efficiently activated to produce a large number of neurons, resulting in improved memory and learning abilities. That is, a summary of the present invention is as follows.
[1] A polynucleotide comprising:
   (A) a nucleic acid sequence of a Plagl2 gene;
   (B) a nucleic acid sequence of an miR-shRNA (microRNA adapted short hairpin RNA) against a Dyrk1a gene; and
   (C) a promoter sequence operably linked to the above nucleic acid sequences.
[2] The polynucleotide according to [1], wherein the Plagl2 gene and the Dyrk1a gene are mammalian genes.
[3] The polynucleotide according to [1] or [2], wherein the promoter is selected from the group consisting of a Hes5 promoter, a GFAP promoter, a Sox2 promoter, and an Lfng promoter.
[4] A vector comprising the polynucleotide according to any one of [1] to [3].
[5] The vector according to [4], wherein the vector is a lentivirus vector, an adeno-associated virus vector, an adenovirus vector, or a plasmid vector.
[6] A pharmaceutical composition comprising the vector according to [4] or [5].
[7] A pharmaceutical composition comprising:
   (a) a vector comprising a nucleic acid sequence of a Plagl2 gene and a promoter sequence operably linked to the nucleic acid sequence of the Plagl2 gene; and
   (b) a vector comprising a nucleic acid sequence of an miR-shRNA (microRNA adapted short hairpin RNA) against a Dyrk1a gene and a promoter sequence operably linked to the nucleic acid sequence of the miR-shRNA, or an siRNA or an miRNA against the Dyrk1a gene.
[8] Cells transformed with the vector according to [4] or [5].
[9] A pharmaceutical composition comprising the cells according to [8].
[10] The pharmaceutical composition according to [6], [7], or [9], wherein the pharmaceutical composition is used for treatment of a neurodegenerative disease or an inner ear disease.
[11] The pharmaceutical composition according to [10], wherein the pharmaceutical composition is administered to a subject by intracerebroventricular injection, intrathecal bolus injection or infusion, intraganglionic injection, intraneural injection, subcutaneous injection, or intratympanic injection.
[12] A method for screening for a substance for treatment of a neurodegenerative disease or an inner ear disease, comprising:
   (1) a step of causing a test substance to act on neural stem cells or inner ear supporting cells to measure expression of a Plagl2 gene and a Dyrk1a gene; and
   (2-1) a step of selecting a test substance having an ability to enhance Plagl2 gene expression and an ability to suppress Dyrk1a gene expression from a result of the step (1) or
   (2-2) a step of selecting a test substance having an ability to enhance Plagl2 gene expression, and a test substance having an ability to suppress Dyrk1a gene expression from the result of the step (1).

### Advantageous Effects of Invention

According to the present invention, by introducing a specific gene set into an adult brain or an aged brain, endogenous neural stem cells can be efficiently activated and proliferated to produce a large number of neurons. The specific gene set can cause neural stem cells in the aged brain to revert to their adolescent or younger state and can produce a large number of neurons, resulting in improved memory and learning abilities. In addition, a therapeutic effect thereof on a neurodegenerative disease such as Alzheimer's disease can also be expected, and endogenous neural stem cells can be efficiently activated to produce a large number of neurons, resulting in improved memory and learning abilities. Further, the specific gene set of the present invention can differentiate not only brain neural stem cells but also supporting cells in the inner ear into hair cells, and it is suggested that the specific gene set is also effective in the treatment of an inner ear disease.

### BRIEF DESCRIPTION OF DRAWINGS

[Fig. 1] Fig. 1 is a diagram showing a step of screening for a gene that contributes to NSC (neural stem cell) activation.
[Fig. 2] Fig. 2 is a diagram showing results of in vitro screening for an NSC-activating gene by overexpression of genes highly expressed in an embryo.
[Fig. 3] Fig. 3 is a diagram showing results of in vivo screening for an NSC-activating gene by overexpression of genes highly expressed in an embryo.
[Fig. 4-1] Fig. 4-1 is a diagram showing results of in vitro screening for an NSC-activating gene by knocking down genes highly expressed in an adult.
[Fig. 4-2] Fig. 4-2 is a diagram showing results of in vitro screening for an NSC-activating gene by knocking down genes highly expressed in an adult.
[Fig. 5] Fig. 5 is a diagram showing results of in vivo screening for an NSC-activating gene.
[Fig. 6] Fig. 6 is a diagram showing results of in vivo screening for an NSC-activating gene.
[Fig. 7] Fig. 7 is a diagram showing long-term activation of neurogenesis in aged mouse brains by iPaD. The combination of Plagl2 overexpression and Dyrk1a knockdown was termed "iPaD (inducing Plagl2 and anti-Dyrk1a)."
[Fig. 8] Fig. 8 is a diagram showing long-term activation of neurogenesis in aged mouse brains by iPaD.
[Fig. 9-1] Fig. 9-1 is a diagram showing the decline in neurogenic ability with aging (wild-type hippocampus).
[Fig. 9-2] Fig. 9-2 is a diagram showing the decline in neurogenic ability with aging and the rejuvenation of aged NSCs by iPaD.
[Fig. 10-1] Fig. 10-1 is a diagram showing the schedule of a Barnes maze test.
[Fig. 10-2] Fig. 10-2 is a diagram showing improving effects of iPaD on cognitive functions of aged mice (Barnes maze test).
[Fig. 10-3] Fig. 10-3 is a diagram showing an improving effect of iPaD on a cognitive function of aging mice (Barnes maze test).
[Fig. 11] Fig. 11 is a diagram showing changes with age in months in the number of MCM2-positive cells in the hippocampal dentate gyrus of Alzheimer's disease model mice (5 × FAD mice) and normal mice (wild-type littermate mice).
[Fig. 12] Fig. 12 is a diagram showing changes with age in months in the number of DCX-positive cells in the hippocampal dentate gyrus of Alzheimer's disease model mice (5 × FAD mice) and normal mice (wild-type littermate mice).
[Fig. 13] Fig. 13 is a diagram showing an age-related increase in the number of amyloid β plaques in the hippocampal dentate gyrus of Alzheimer's disease model mice (5 × FAD mice).
[Fig. 14] Fig. 14 is a diagram showing results of comparing a cognitive function between Alzheimer's disease model mice (5 × FAD mice) and normal mice (wild-type littermate mice) (Barnes circular maze test).
[Fig. 15] Fig. 15 is a diagram showing results of comparing a cognitive function between Alzheimer's disease model mice (5 × FAD mice) and normal mice (wild-type littermate mice) (contextual fear conditioning test).
[Fig. 16] Fig. 16 is a diagram showing an improving effect of iPaD on Alzheimer's disease (number of MCM2-positive cells).
[Fig. 17] Fig. 17 is a diagram showing an improving effect of iPaD on Alzheimer's disease (number of DCX-positive cells).
[Fig. 18] Fig. 18 is a diagram showing an improving effect of iPaD on Alzheimer's disease (number of amyloid β plaques).
[Fig. 19] Fig. 19 is a diagram showing an improving effect of iPaD on a cognitive function of Alzheimer's disease model mice (5 × FAD mice) (Barnes circular maze test).
[Fig. 20] Fig. 20 is a diagram showing an improving effect of iPaD on a cognitive function of Alzheimer's disease model mice (5 × FAD mice) (contextual fear conditioning test).
[Fig. 21] Fig. 21 is a diagram showing activation of supporting cells and regeneration of hair cells in the inner ear by iPaD.

### DESCRIPTION OF EMBODIMENTS

Hereinafter, the present invention will be described in detail. Herein, a molecular biological technique can be carried out by a method disclosed in a general laboratory book known to those skilled in the art or a method based thereon, unless otherwise specified. In addition, the terms used herein are interpreted as having a meaning commonly used in the technical field, unless otherwise specified.

According to the present disclosure, by forcibly expressing a gene that is highly expressed in an embryo and low expressed in adult neural stem cells, and suppressing expression of a gene that is low expressed in an embryo and highly expressed in adult neural stem cells, in target cells such as neural stem cells, these cells can be efficiently activated and proliferated. That is, in the present application, provided are a polynucleotide, a vector, and transformed cells that include a specific gene set that can efficiently activate and proliferate endogenous neural stem cells or the like in an adult brain, an aged brain, a diseased brain, or the like to produce a large number of neurons, a pharmaceutical composition including the polynucleotide, the vector, or the transformed cells, a method for treating a neurodegenerative disease or the like using the polynucleotide, the vector, the transformed cells, or the pharmaceutical composition, and the like.

### <Polynucleotide>

A first embodiment of the polynucleotide of the present disclosure is a polynucleotide including: (A) a nucleic acid sequence of a gene that is highly expressed in an embryo and low expressed in adult neural stem cells; (B) a nucleic acid sequence that suppresses expression of a gene that is low expressed in an embryo and highly expressed in adult neural stem cells (for example, nucleic acid sequence of miR-shRNA (microRNA adapted short hairpin RNA) against the gene); and (C) a promoter sequence operably linked to the nucleic acid sequences, to be introduced into target cells such as neural stem cells.

A second embodiment of the polynucleotide of the present disclosure is a combination of a polynucleotide including (a) a nucleic acid sequence of a gene that is highly expressed in an embryo and low expressed in adult neural stem cells, and a promoter sequence operably linked to the nucleic acid sequence of the gene, and a polynucleotide including (b1) a nucleic acid sequence that suppresses expression of a gene that is low expressed in an embryo and highly expressed in adult neural stem cells (for example, nucleic acid sequence of miR-shRNA (microRNA adapted short hairpin RNA) against the gene), and a promoter sequence operably linked to the nucleic acid sequence that suppresses expression of the gene (for example, nucleic acid sequence of miR-shRNA), to be introduced into target cells such as neural stem cells.

A third embodiment of the polynucleotide of the present disclosure is a combination of a polynucleotide including (a) a nucleic acid sequence of a gene that is highly expressed in an embryo and low expressed in adult neural stem cells, and a promoter sequence operably linked to the nucleic acid sequence of the gene, and the gene and (b2) a nucleic acid sequence that suppresses expression of a gene that is low expressed in an embryo and highly expressed in adult neural stem cells (for example, polynucleotide of siRNA or miRNA against the gene), to be introduced into target cells such as neural stem cells.

As used herein, a "polynucleotide" is used interchangeably with a "nucleic acid", a "gene," or a "nucleic acid molecule" and means a polymer of a nucleotide. As used herein, the term "nucleotide sequence" is used interchangeably with a "nucleic acid sequence" or a "base sequence" and is expressed as a sequence of deoxyribonucleotides (abbreviated as A, G, C, and T). For example, "a polynucleotide including the nucleotide sequence of SEQ ID NO: 1 or a fragment thereof" means a polynucleotide including the sequence represented by each deoxynucleotide A, G, C, and/or T of SEQ ID NO: 1 or a fragment portion thereof.

Examples of target cells into which the polynucleotide of the present disclosure is introduced include tissue stem cells (somatic stem cells) such as neural stem cells, inner ear supporting cells, hematopoietic stem cells, mesenchymal stem cells, skeletal muscle stem cells, or dental pulp stem cells, retinal Muller glial cells, and tissue progenitor cells, and among these, neural stem cells and inner ear supporting cells are preferable from the viewpoint of activation and proliferation being promoted by introducing the polynucleotide of the present disclosure.

### (Regarding first embodiment)

The first embodiment of the polynucleotide of the present disclosure to be introduced into target cells such as neural stem cells is a polynucleotide including: (A) a nucleic acid sequence of a gene that is highly expressed in an embryo and low expressed in adult neural stem cells; (B) a nucleic acid sequence that suppresses expression of a gene that is low expressed in an embryo and highly expressed in adult neural stem cells (for example, nucleic acid sequence of miR-shRNA (microRNA adapted short hairpin RNA) against the gene); and (C) a promoter sequence operably linked to the nucleic acid sequences.

### [(A) Nucleic acid sequence of gene that is highly expressed in embryo and low expressed in adult neural stem cells]

The nucleic acid sequence (A) is not particularly limited as long as it is a nucleic acid sequence of a gene that is highly expressed in an embryo and low expressed in adult neural stem cells, and above all, the nucleic acid sequence is preferably a nucleic acid sequence of a gene that promotes proliferation and activation of adult neural stem cells by overexpression thereof in the adult neural stem cells; and specific examples thereof include nucleic acid sequences of Gsx2, Dmrt3, Cdk4, Plagl2, Sox21, Ascl1, Tgif2, Plagl1, Hmga2, and the like. Among these, the nucleic acid sequence of the Plagl2 gene is more preferable from the viewpoint of a superior ability to proliferate and activate adult neural stem cells. The Plagl2 gene (Pleiomorphic adenoma gene like-2) is a gene encoding a transcription factor PLAGL2 having a C2H2 zinc finger region. The Plagl2 gene is also referred to as ZNF900. PLAGL2 is known to function as a positive regulator of transcription and be localized in the nucleus. As described in detail in the Examples, in the present disclosure, PLAGL2 was found as one of the nuclear factors differentially expressed by comparing the transcriptomes (DDBJ BioProject Accession: PRJDB9010) of G1/G0 NSCs derived from the ganglionic eminence and the dorsal cortex of E14 mouse embryos with those of NSCs mostly quiescent derived from LV-SVZ (SAMD00192826) and DG-SGZ (SAMD00192827) in 2- to 3-month-old mice.

In the present disclosure, a gene that is highly expressed in an embryo and low expressed in adult neural stem cells, such as the Plagl2 gene, are derived from mammals such as a human, a monkey, a pig, a horse, a cow, a rabbit, a sheep, a goat, a cat, a dog, or a guinea pig, and among these, the gene is preferably derived from a human, a monkey, or a mouse, and more preferably derived from a human.

In the present disclosure, the nucleic acid sequence of a gene that is highly expressed in an embryo and low expressed in adult neural stem cells, such as the Plagl2 gene, includes a coding region (CDS) within an mRNA sequence of the gene. As the nucleic acid sequence of the gene, a sequence known as a coding region (CDS) of an mRNA sequence of each of the genes derived from the above mammals can be adopted, and for example, a nucleotide sequence registered in the GenBank nucleotide sequence database or the like can be used. The nucleic acid sequence of the Plagl2 gene in the present disclosure is a nucleic acid sequence which is the same (SEQ ID NO: 1) as the nucleotide sequence registered in the GenBank nucleotide sequence database or the like, or has a homology of 80% or more, preferably 90% or more, more preferably 95% or more, or further preferably 98% or more, and particularly preferably 99% or more therewith, and in which the peptide encoded has the transcription factor activity as PLAGL2. Examples of a most preferable nucleic acid sequence of the Plagl2 gene in the present disclosure include the sequence of SEQ ID NO: 1.

### [(B) Nucleic acid sequence that suppresses expression of gene that is low expressed in an embryo and highly expressed in adult neural stem cells

The nucleic acid sequence (B) is not particularly limited as long as it is a nucleic acid sequence that suppresses expression of a gene that is low expressed in an embryo and highly expressed in adult neural stem cells, and such a gene that is low expressed in an embryo and highly expressed in adult neural stem cells is preferably a gene that, when knocked down, can efficiently activate quiescent neural stem cells; and examples thereof include Cdkn1a, Prkcz, Dyrk1a, Zbtb7a, Dusp22, Cidea, Rasd1, Nr4a1, Nfe2l2, Stat6, and Tsc22d3, and among these, Cdkn1a, Prkcz, Dyrk1a, and Zbtb7a are more preferable, and Dyrk1a is further preferable. The Dyrk1a gene encodes an enzyme that phosphorylates serine/threonine and tyrosine, and is a gene that is thought to be possibly involved in the development of Down's syndrome caused by trisomy of chromosome 21. As described in detail in the Examples, in the present disclosure, the Dyrk1a gene was found as one of the above 11 genes that, when knocked down, effectively activated quiescent NSCs in target neural stem cells.

In the present disclosure, a gene that is low expressed in an embryo and highly expressed in adult neural stem cells, such as the Dyrk1a gene, is derived from mammals such as a human, a monkey, a pig, a horse, a cow, a rabbit, a sheep, a goat, a cat, a dog, or a guinea pig, and among these, the gene is preferably derived from a human, a monkey, or a mouse, and more preferably derived from a human.

In the present disclosure, the nucleic acid sequence of a gene that is low expressed in an embryo and highly expressed in adult neural stem cells, such as the Dyrk1a gene, includes a coding region (CDS) within an mRNA sequence of the gene. As the nucleic acid sequence of the gene, a sequence known as a coding region (CDS) of an mRNA sequence of each of the genes derived from the above mammals can be adopted, and for example, a nucleotide sequence registered in the GenBank nucleotide sequence database or the like can be used. The nucleic acid sequence of the Dyrk1a gene in the present disclosure is a nucleic acid sequence which is the same (SEQ ID NO: 2) as the nucleotide sequence registered in the GenBank nucleotide sequence database or the like, or has a homology of 80% or more, preferably 90% or more, more preferably 95% or more, or further preferably 98% or more, and particularly preferably 99% or more therewith, and in which the peptide encoded has the enzymatic activity as Dyrk1a. Examples of a most preferable nucleic acid sequence of the Dyrk1a gene in the present disclosure include the sequence of SEQ ID NO: 2.

In the present disclosure, the nucleic acid sequence that suppresses expression of a gene that is low expressed in an embryo and highly expressed in adult neural stem cells is not particularly limited as long as it has a nucleic acid sequence that can suppress expression of the gene by a conventionally known method, and examples thereof include an miR-shRNA (microRNA adapted short hairpin RNA) against the gene. The miR-shRNA against the gene is an shRNA (hairpin-shaped RNA sequence used for gene silencing by RNA interference) that can suppress expression of the gene. The shRNA is not particularly limited as long as it has a nucleic acid sequence that can suppress expression of the gene, and can be appropriately designed according to the sequence of the gene. Preferable examples of a nucleic acid sequence of such an shRNA include the nucleic acid sequence of SEQ ID NO: 3, which is the nucleic acid sequence of an miR-shRNA against Dyrk1a, and a nucleic acid sequence that has a homology of 80% or more, preferably 90% or more, more preferably 95% or more, further preferably 98% or more, and particularly preferably 99% or more with the nucleic acid sequence of SEQ ID NO: 3 and that can suppress expression of the Dyrk1a gene can also be adopted as a nucleic acid sequence of an miR-shRNA (microRNA adapted short hairpin RNA) against the Dyrk1a gene in the present disclosure.

### [(C) Promoter sequence operably linked to the nucleic acid sequences]

In the present disclosure, a nucleic acid sequence of a gene that is highly expressed in an embryo and low expressed in adult neural stem cells, and a nucleic acid sequence that suppresses expression of a gene that is low expressed in an embryo and highly expressed in adult neural stem cells (for example, nucleic acid sequence of miR-shRNA (microRNA adapted short hairpin RNA) against the gene) are operably linked to a promoter sequence (C). As the promoter sequence (C) in the present embodiment, a promoter sequence that functions in nervous system cells such as neural stem cells or inner ear supporting cells can be adopted. Examples of such a promoter sequence include the Hes5 promoter (SEQ ID NO: 4) and the GFAP promoter, which function in neural stem cells, and the Sox2 promoter and the Lfng promoter, which function in inner ear supporting cells.

Here, "operably linked" refers to a juxtaposition in which the components described are in a relationship that enables them to function in their intended manner. In one embodiment, this term refers to a functional linkage between a nucleic acid expression control sequence (for example, promoter and/or enhancer) and a target polynucleotide sequence, and the functional linkage may be a direct linkage or an indirect linkage (in which another polynucleotide sequence intervenes therebetween). In the present disclosure, the promoter sequence directs transcription of the target polynucleotide linked.

The polynucleotide including (A) a nucleic acid sequence of a gene that is highly expressed in an embryo and low expressed in adult neural stem cells, (B) a nucleic acid sequence that suppresses expression of a gene that is low expressed in an embryo and highly expressed in adult neural stem cells (for example, nucleic acid sequence of miR-shRNA (microRNA adapted short hairpin RNA) against the gene), and (C) a promoter sequence operably linked to the nucleic acid sequences according to the present embodiment is preferably a polynucleotide including (A) a nucleic acid sequence of the Plagl2 gene, (B) a nucleic acid sequence that suppresses expression of the Dyrk1a gene (for example, nucleic acid sequence of miR-shRNA (microRNA adapted short hairpin RNA) against the Dyrk1a gene), and (C) a promoter sequence operably linked to the nucleic acid sequences, and more preferable examples thereof include the polynucleotide of SEQ ID NO: 5, and a polynucleotide that is derived from the polynucleotide of SEQ ID NO: 5 by deletion, substitution, insertion, and/or addition of one or more (for example, 1 to 50, 1 to 40, 1 to 30, 1 to 25, 1 to 20, 1 to 15, 1 to 10, 1 to 9, 1 to 8, 1 to 7, 1 to 6, 1 to 5, 1 to 4, 1 to 3, 1 or 2, or 1) nucleotides and that, when introduced into a vector to transform target cells, can forcibly express the Plagl2 gene and knock down the Dyrk1a gene. A combinations of two or more of these deletion, substitution, insertion, and addition may be included at the same time. In general, the smaller the number of deletions, substitutions, insertions, and/or additions of the nucleotides, the more preferable. In addition, in the present invention, preferable polynucleotides include, for example, a polynucleotide that is hybridizable under a stringent hybridization condition to a sequence complementary to SEQ ID NO: 5.

Here, the "stringent condition" may be any of a low stringent condition, a moderately stringent condition, and a highly stringent condition. The "low stringent condition" is, for example, conditions of 5 × SSC, 5 × Denhardt's solution, 0.5% SDS, 50% formamide, and 32°C. In addition, the "moderately stringent condition" is, for example, conditions of 5 × SSC, 5 × Denhardt's solution, 0.5% SDS, 50% formamide, and 42°C. The "highly stringent condition" is, for example, conditions of 5 × SSC, 5 × Denhardt's solution, 0.5% SDS, 50% formamide, and 50°C. Under these conditions, it can be expected that the higher the temperature, the more efficiently a DNA having a higher homology can be obtained. However, a plurality of factors such as temperature, probe concentration, probe length, ionic strength, time, and salt concentration can be considered as factors that affect the stringency of hybridization, and those skilled in the art can appropriately select these factors to achieve similar stringency.

The polynucleotide of the present embodiment may further include a known sequence such as an enhancer sequence, a Kozak sequence, and an appropriate polyadenylation signal sequence that assist in transcription of mRNA, translation into a protein, or the like.

The method for introducing the polynucleotide of the present embodiment into target cells is not particularly limited, and examples thereof include using a vector such as a virus, a plasmid, or an artificial chromosome, or a technique such as lipofection, a liposome, or microinjection. When the polynucleotide of the present embodiment is introduced into target neural stem cells or the like to transform the same, it is possible to forcibly express a gene that is highly expressed in an embryo and low expressed in adult neural stem cells, such as the Plagl2 gene, and knock down a gene that is low expressed in an embryo and highly expressed in adult neural stem cells, such as the Dyrk1a gene. The target cells (neural stem cells or the like) thus transformed can be activated and proliferated to differentiate to fully exhibit cellular functions, such as producing a large number of neurons. The polynucleotide of the present embodiment can be suitably used for endogenous neural stem cells in an adult brain, an aged brain, a diseased brain, or the like, supporting cells present in the inner ear, or the like.

### (Regarding second embodiment)

The second embodiment of the polynucleotide of the present disclosure to be introduced into target cells such as neural stem cells is a combination of a polynucleotide including (a) a nucleic acid sequence of a gene that is highly expressed in an embryo and low expressed in adult neural stem cells, and a promoter sequence operably linked to the nucleic acid sequence of the gene, and a polynucleotide including (b1) a nucleic acid sequence that suppresses expression of a gene that is low expressed in an embryo and highly expressed in adult neural stem cells (for example, nucleic acid sequence of miR-shRNA (microRNA adapted short hairpin RNA) against the gene), and a promoter sequence operably linked to the nucleic acid sequence that suppresses expression of the gene. The combination of polynucleotides according to the present embodiment is used to, for example, introduce a gene that is highly expressed in an embryo and low expressed in adult neural stem cells and a nucleic acid sequence that suppresses expression of a gene that is low expressed in an embryo and highly expressed in adult neural stem cells (for example, miR-shRNA against the Dyrk1a gene) into separate vectors to separately transform target cells such as neural stem cells.

To (a) a nucleic acid sequence of a gene that is highly expressed in an embryo and low expressed in adult neural stem cells in the present embodiment, the description of (A) a nucleic acid sequence of a gene that is highly expressed in an embryo and highly expressed in adult neural stem cells in the first embodiment described above can be directly applied. In addition, to a promoter sequence operably linked to the nucleic acid sequence of the gene, the description of (C) a promoter sequence operably linked to the nucleic acid sequence in the first embodiment can also be applied, and specific examples thereof include the Hes5 promoter (SEQ ID NO: 4) and the GFAP promoter, which function in neural stem cells, and the Sox2 promoter and the Lfng promoter, which function in inner ear supporting cells.

The polynucleotide including (a) a nucleic acid sequence of a gene that is highly expressed in an embryo and low expressed in adult neural stem cells and a promoter sequence operably linked to the nucleic acid sequence of the gene in the present embodiment is preferably a polynucleotide including (a) a nucleic acid sequence of the Plagl2 gene and a promoter sequence operably linked to the nucleic acid sequence of the Plagl2 gene, and more preferable examples thereof include a polynucleotide including the nucleic acid sequence of SEQ ID NO: 6. This polynucleotide may further include a known sequence such as an enhancer sequence, a Kozak sequence, and an appropriate polyadenylation signal sequence that assist in transcription of mRNA, translation into a protein, or the like.

To (b1) a nucleic acid sequence that suppresses expression of a gene that is low expressed in an embryo and highly expressed in adult neural stem cells (for example, nucleic acid sequence of miR-shRNA (microRNA adapted short hairpin RNA) against the gene) in the present embodiment, the description of (B) a nucleic acid sequence of a gene that is low expressed in an embryo and highly expressed in adult neural stem cells in the first embodiment described above can be directly applied. In addition, to a promoter sequence operably linked to the nucleic acid sequence, the description of (C) a promoter sequence operably linked to the nucleic acid sequences in the first embodiment can be applied, and specific examples thereof include the Hes5 promoter (SEQ ID NO: 4) and the GFAP promoter, which function in neural stem cells, and the Sox2 promoter and the Lfng promoter, which function in inner ear supporting cells.

The polynucleotide including (b1) a nucleic acid sequence that suppresses expression of a gene that is low expressed in an embryo and highly expressed in adult neural stem cells (for example, nucleic acid sequence of miR-shRNA (microRNA adapted short hairpin RNA) against the Dyrk1a gene) and a promoter sequence operably linked to the nucleic acid sequence in the present embodiment is preferably a polynucleotide including (b 1) a nucleic acid sequence of an miR-shRNA (microRNA adapted short hairpin RNA) against the Dyrk1a gene and a promoter sequence operably linked to the nucleic acid sequence of the miR-shRNA, and more preferable examples thereof include a polynucleotide including the nucleic acid sequence of SEQ ID NO: 7. This polynucleotide may further include a known sequence such as an enhancer sequence, a Kozak sequence, and an appropriate polyadenylation signal sequence that assist in transcription of mRNA, translation into a protein, or the like.

The method for introducing the polynucleotide of the present embodiment into target cells is not particularly limited, and examples thereof include using a vector such as a virus, a plasmid, or an artificial chromosome, or a technique such as lipofection, a liposome, or microinjection. The two types of polynucleotides of the present embodiment can be each independently introduced into target cells. For example, when the two types are incorporated into separate vectors and introduced into target neural stem cells or the like to transform the same, it is possible to forcibly express a gene that is highly expressed in an embryo and low expressed in adult neural stem cells and knock down a gene that is low expressed in an embryo and highly expressed in adult neural stem cells. The target cells (neural stem cells or the like) thus transformed can be activated and proliferated to fully exhibit cellular functions, such as producing a large number of neurons. The polynucleotides of the present embodiment can be suitably used for endogenous neural stem cells in an adult brain, an aged brain, a diseased brain, or the like, supporting cells present in the inner ear, or the like.

### (Regarding third embodiment)

The third embodiment of the polynucleotide of the present disclosure to be introduced into target cells such as neural stem cells is a combination of a polynucleotide including (a) a nucleic acid sequence of a gene that is highly expressed in an embryo and low expressed in adult neural stem cells, and a promoter sequence operably linked to the nucleic acid sequence of the gene, and (b2) a polynucleotide of an siRNA or an miRNA against a gene that is low expressed in an embryo and highly expressed in adult neural stem cells. In the present embodiment, the polynucleotide (a) is introduced into a vector, and the polynucleotide (siRNA or miRNA) (b2) is used together with a transfection reagent and the like to transform target cells.

To (a) a nucleic acid sequence of a gene that is highly expressed in an embryo and low expressed in adult neural stem cells in the present embodiment, the description of (A) a nucleic acid sequence of a gene that is highly expressed in an embryo and low expressed in adult neural stem cells in the first embodiment described above can be directly applied. In addition, to a promoter sequence operably linked to the nucleic acid sequence of the gene, the description of (C) a promoter sequence operably linked to the nucleic acid sequences in the first embodiment can be applied, and specific examples thereof include the Hes5 promoter (SEQ ID NO: 4) and the GFAP promoter, which function in neural stem cells, and the Sox2 promoter and the Lfng promoter, which function in inner ear supporting cells.

The polynucleotide including (a) a nucleic acid sequence of a gene that is highly expressed in an embryo and low expressed in adult neural stem cells and a promoter sequence operably linked to the nucleic acid sequence of the gene in the present embodiment is preferably a polynucleotide including (a) a nucleic acid sequence of the Plagl2 gene and a promoter sequence operably linked to the nucleic acid sequence of the Plagl2 gene, and more preferable examples thereof include a polynucleotide including the nucleic acid sequence of SEQ ID NO: 6.

The polynucleotide (b2) in the present embodiment is not particularly limited as long as it is an siRNA or an miRNA of a gene that is low expressed in an embryo and highly expressed in adult neural stem cells and having a sequence that can suppress expression of (knock down) the gene. The siRNA or miRNA of the gene can be designed from the sequence of the gene. For example, an siRNA or an miRNA of the Dyrk1a gene can be designed from the sequence of the Dyrk1a gene, and specific examples thereof include the polynucleotide of SEQ ID NO: 8.

The method for introducing the polynucleotide of the present embodiment into target cells is not particularly limited, and examples thereof include using a vector such as a virus, a plasmid, or an artificial chromosome, or a technique such as lipofection, a liposome, or microinjection. For example, target cells (neural stem cells or the like) can be transformed with a vector into which the polynucleotide (a) of the present embodiment has been introduced to forcibly express the Plagl2 gene or the like, and further, the target cells can be transfected with an siRNA or an miRNA of the Dyrk1a gene or the like as the polynucleotide (b2) to suppress expression of the Dyrk1a gene or the like. A transfection method conventionally known to those skilled in the art can be used. The target cells (neural stem cells or the like) thus transformed can be activated and proliferated to fully exhibit cellular functions, such as producing a large number of neurons. The polynucleotide of the present embodiment can be suitably used for endogenous neural stem cells in an adult brain, an aged brain, a diseased brain, or the like, supporting cells present in the inner ear, or the like.

### <Vector>

The disclosure also includes a vector including the polynucleotide of the disclosure described above. To the polynucleotide of the present disclosure included in the vector of the present disclosure, the description of the polynucleotide section described above can be directly applied.

In the present disclosure, the vector used to forcibly express a gene that is highly expressed in an embryo and low expressed in adult neural stem cells (Plagl2 gene or the like), and/or suppress the expression of (knock down) a gene that is low expressed in an embryo and highly expressed in adult neural stem cells (Dyrk1a gene or the like) in target cells is not particularly limited as long as it is a vector that can efficiently deliver the gene to target cells (neural stem cells, inner ear supporting cells, or the like). Examples of such a vector include a virus vector such as a lentivirus vector, an adeno-associated virus vector, an adenovirus vector, a herpes virus vector (for example, herpes simplex virus vector), a pox virus vector, a baculovirus vector, a papilloma virus vector, or a papovavirus vector (for example, SV40); a plasmid vector; a phagemid vector; a cosmid vector; and a bacteriophage such as lambda phage or M13 phage. Among these, a lentivirus vector, an adeno-associated virus vector, an adenovirus vector, and a plasmid vector are preferable, a lentivirus vector and an adeno-associated virus vector are more preferable, and a lentivirus vector is further preferable.

Examples of a specific expression vector include pLenti4/V5-DEST(TM), pLenti6/V5-DEST(TM), and pLenti6.2/V5-GW/lacZ (Invitrogen) for lentivirus-mediated gene transfer and expression in mammalian cells; and pClneo Vector (Promega) for expression in mammalian cells.

The vector can include a control sequence such as a promoter, an enhancer, a ribosome binding sequence, a terminator, or a polyadenylation site so that the target gene can be expressed. In addition, if necessary, the vector can include a selection marker sequence such as a drug resistance gene (for example, kanamycin resistance gene, ampicillin resistance gene, or puromycin resistance gene), a thymidine kinase gene, or a diphtheria toxin gene, a reporter gene sequence such as mCherry (red fluorescent protein), a green fluorescent protein (GFP), β-glucuronidase (GUS), or FLAG, or the like.

### <Pharmaceutical composition>

The present disclosure also includes a pharmaceutical composition containing the vector of the present disclosure described above. In addition, the present disclosure also includes a pharmaceutical composition containing (a) a vector including a nucleic acid sequence of a gene that is highly expressed in an embryo and low expressed in adult neural stem cells (Plagl2 gene or the like), and a promoter sequence operably linked to the nucleic acid sequence of the gene, and (b) a vector including a nucleic acid sequence that suppresses expression of a gene that is low expressed in an embryo and highly expressed in adult neural stem cells (Dyrk1a gene or the like) (for example, nucleic acid sequence of miR-shRNA (microRNA adapted short hairpin RNA) against the gene), and a promoter sequence operably linked to the nucleic acid sequence that suppresses expression of the gene, or an siRNA or an miRNA against the gene. Further, the present disclosure also includes a pharmaceutical composition containing the transformed cells of the present disclosure. The pharmaceutical compositions of the present disclosure may contain at least one selected from the group consisting of a pharmaceutically acceptable adjuvant, excipient, carrier, and diluent. To the vectors and transformed cells of the present disclosure, the descriptions in their respective sections can be applied. These pharmaceutical compositions of the present disclosure are used for the treatment of a neurodegenerative disease or an inner ear disease.

The present disclosure also includes a pharmaceutical composition for the treatment of a neurodegenerative disease or an inner ear disease containing a compound having the ability to enhance expression of a gene that is highly expressed in an embryo and low expressed in adult neural stem cells (Plagl2 gene or the like) and the ability to suppress expression of a gene that is low expressed in an embryo and highly expressed in adult neural stem cells (Dyrk1a gene or the like) or a prodrug thereof or a pharmaceutically acceptable salt thereof as an active ingredient. Further, the present disclosure also includes a pharmaceutical composition for the treatment of a neurodegenerative disease or an inner ear disease containing a compound having the ability to enhance expression of a gene that is highly expressed in an embryo and low expressed in adult neural stem cells (Plagl2 gene or the like) or a prodrug thereof or a pharmaceutically acceptable salt thereof, and a compound having the ability to suppress expression of a gene that is low expressed in an embryo and highly expressed in adult neural stem cells (Dyrk1a gene or the like) or a prodrug thereof or a pharmaceutically acceptable salt thereof as active ingredients.

The expression of the gene that is highly expressed in an embryo and low expressed in adult neural stem cells (Plagl2 gene or the like) and the expression of the gene that is low expressed in an embryo and highly expressed in adult neural stem cells (Dyrk1a gene or the like) are preferably the expressions of the genes in neural stem cells or inner ear supporting cells. In addition, the above compounds, or prodrugs thereof, or pharmaceutically acceptable salts thereof may be any of low molecular weight compounds, middle molecular weight compounds, and high molecular weight compounds.

The pharmaceutical composition of the present disclosure is administered to a subject by intracerebroventricular injection, intrathecal bolus injection or infusion, intraganglionic injection, intraneural injection, subcutaneous injection, or intratympanic injection.

The dosage of the pharmaceutical composition of the present disclosure is not particularly limited, and an appropriate dosage can be selected according to various conditions such as the type of the disease, the age and the symptom of the patient, the administration route, the purpose of treatment, and the presence or absence of a concomitant agent.

### <Treatment method>

The present disclosure also includes a method for treating a neurodegenerative disease including forcibly expressing a gene that is highly expressed in an embryo and low expressed in adult neural stem cells (Plagl2 gene or the like) and suppressing expression of a gene that is low expressed in an embryo and highly expressed in adult neural stem cells (Dyrk1a gene or the like) in neural stem cells. In addition, the present disclosure also includes a method for treating an inner ear disease including forcibly expressing a gene that is highly expressed in an embryo and low expressed (Plagl2 gene or the like) and suppressing expression of a gene that is low expressed in an embryo and highly expressed in adult neural stem cells (Dyrk1a gene or the like) in inner ear supporting cells. Examples of a method for forcibly expressing a gene that is highly expressed in an embryo and low expressed (Plagl2 gene or the like) and suppressing expression of a gene that is low expressed in an embryo and highly expressed in adult neural stem cells (Dyrk1a gene or the like) in target cells such as inner ear supporting cells include a method using the polynucleotide, vector, the transformed cells, and the pharmaceutical composition according to the present disclosure, and to the descriptions thereof, the descriptions in the Polynucleotide, Vector, Pharmaceutical composition sections described above, respectively, can be applied.

### transformed cells>

The disclosure also includes target cells into which the polynucleotide of the disclosure described above has been introduced. For example, cells transformed with the vector of the disclosure described above, cells obtained by transformation with the vector of the disclosure, transfection with the polynucleotide of the disclosure, or the like are also included in the disclosure. The transformed cells of the present disclosure can be prepared in vitro and administered to a subject in need of treatment. At that time, the transformed cells of the present disclosure can be administered as a pharmaceutical composition together with a pharmaceutically acceptable adjuvant, excipient, carrier, or diluent.

Further, the present disclosure also includes cells transformed by treatment with a compound having the ability to enhance expression of a gene that is highly expressed in an embryo and low expressed in adult neural stem cells (Plagl2 gene or the like) and the ability to suppress expression of a gene that is low expressed in an embryo and highly expressed in adult neural stem cells (Dyrk1a gene or the like) or a prodrug thereof or a pharmaceutically acceptable salt thereof, and cells transformed by treatment with a compound having the ability to enhance expression of a gene that is highly expressed in an embryo and low expressed in adult neural stem cells (Plagl2 gene or the like) or a prodrug thereof or a pharmaceutically acceptable salt thereof, and a compound having the ability to suppress expression of a gene that is low expressed in an embryo and highly expressed in adult neural stem cells (Dyrk1a gene or the like) or a prodrug thereof or a pharmaceutically acceptable salt thereof. Such cells are preferably cells obtained by the above transformation of the neural stem cells or inner ear supporting cells, and the above compounds, or prodrugs thereof, or pharmaceutically acceptable salts thereof may be any of low molecular weight compounds, middle molecular weight compounds, and high molecular weight compounds.

### <Method for screening for substance for treatment of neurodegenerative disease or inner ear disease>

The present disclosure also includes a method for screening for a substance for treatment of a neurodegenerative disease or an inner ear disease, including:
(1) a step of causing a test substance to act on neural stem cells or inner ear supporting cells to measure expression of a gene that is highly expressed in an embryo and low expressed in adult neural stem cells (Plagl2 gene or the like) and a gene that is low expressed in an embryo and highly expressed in adult neural stem cells (Dyrk1a gene or the like); and
(2-1) a step of selecting a test substance that has an ability to enhance expression of a gene that is highly expressed in an embryo and low expressed in adult neural stem cells (Plagl2 gene or the like) and an ability to suppress expression of a gene that is low expressed in an embryo and highly expressed in adult neural stem cells (Dyrk1a gene or the like) from a result of the step (1) or
(2-2) a step of selecting a test substance that has an ability to enhance expression of a gene that is highly expressed in an embryo and low expressed in adult neural stem cells (Plagl2 gene or the like) and a test substance that has an ability to suppress expression of a gene that is low expressed in an embryo and highly expressed in adult neural stem cells (Dyrk1a gene or the like) from the result of the step (1).

The substance(s) selected by the screening method of the present disclosure can efficiently activate and proliferate neural stem cells to produce a large number of neurons. Because of this, the substance(s) can cause neural stem cells in an aged brain to revert to their adolescent or younger state and can be expected to produce a large number of neurons, resulting in improved memory and learning abilities. In addition, a therapeutic effect thereof on a neurodegenerative disease such as Alzheimer's disease can also be expected, and endogenous neural stem cells can be efficiently activated to produce a large number of neurons, resulting in improved memory and learning abilities. Further, the substance(s) selected by the screening method of the present invention can differentiate not only brain neural stem cells but also supporting cells in the inner ear into hair cells, and it is expected that the screening method is also effective in the treatment of an inner ear disease.

### EXAMPLES

The present disclosure will be specifically described with reference to the following Examples, but the present disclosure should not be construed as limited by these Examples.

### 1. Screening for neural stem cell (NSC)-activating gene

The present inventors reasoned that it was necessary to alter expression of a plurality of genes in order to activate and proliferate aged NSCs. Therefore, most adult NSCs are quiescent, and thus the transcriptomes (DDBJ BioProject Accession: PRJDB9010) of G1/G0 NSCs derived from the ganglionic eminence and the dorsal cortex of E14 mouse embryos was compared with those of NSCs mostly quiescent derived from LV-SVZ (SAMD00192826) and DG-SGZ (SAMD00192827) in 2- to 3-month-old mice to identify nuclear factors differentially expressed and known modulators therefor.

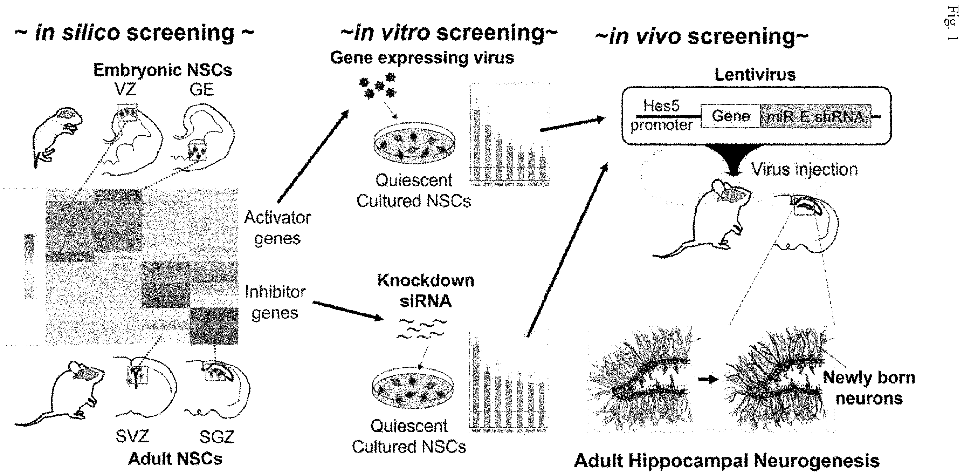

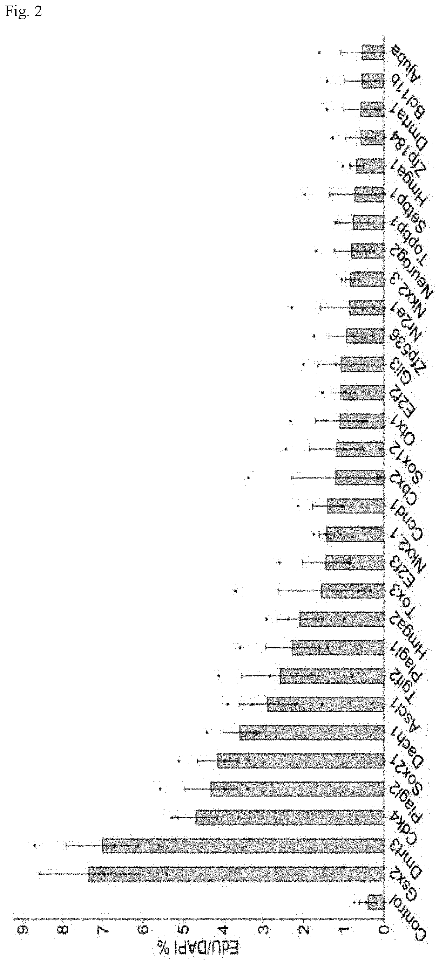

First, genes that were expressed at a high level in an embryo and low expressed in adult NSCs ("embryonic-high") were identified. Next, these genes were overexpressed in vitro in NSCs maintained quiescent (EdU⁻) by bFGF and BMP (Fig. 1). After two days, the proportions of proliferating cells that had incorporated EdU were measured to identify genes capable of proliferating and activating NSCs. In the present study, each of the top 80 genes expressed at a high level in an embryo was overexpressed in quiescent NSCs in vitro, and proliferation and activation of NSCs were monitored. Results thereof are shown in Fig. 2. Among the genes tested, Gsx2, Dmrt3, Cdk4, Plagl2, Sox21, Ascl1, Tgif2, Plagl1, and Hmga2 efficiently activated quiescent NSCs.

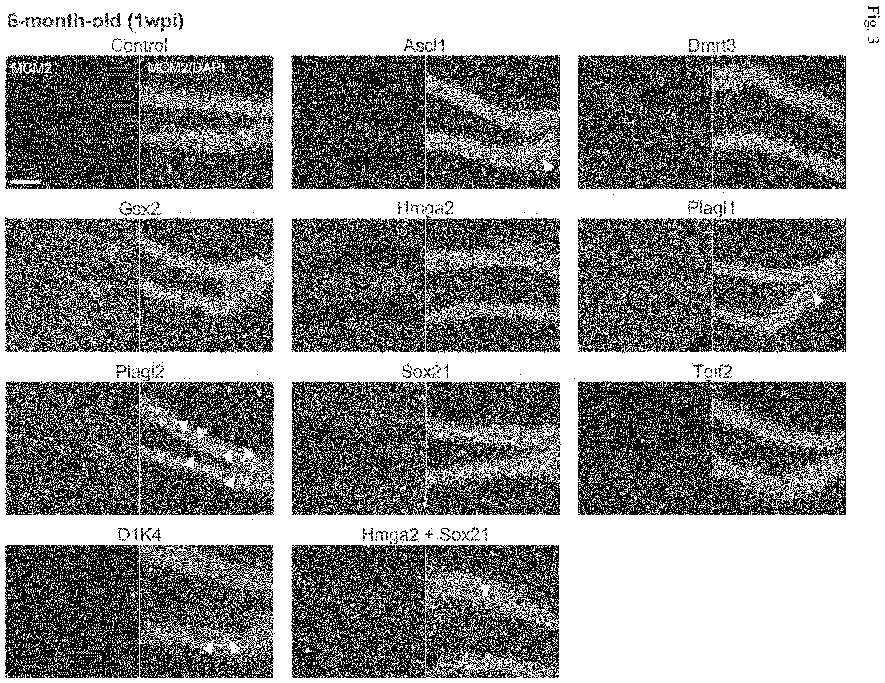

Further, in vivo studies were carried out on Gsx2, Dmrt3, Cdk4, Plagl2, Sox21, Ascl1, Tgif2, Plagl1, and Hmga2. These nine genes were individually cloned into a lentivirus under the control of the Hes5 promoter (SEQ ID NO: 9), which operates specifically in NSCs and astrocytes. Each lentivirus was injected into the hippocampal dentate gyrus of 6-month-old mice, and brain sections were investigated after one week (Fig. 3). The combination of Cdk4 and cyclin D1 (D1K4) is known to activate NSCs in the adult brain, and thus these were used as positive controls. Among the genes tested, Plagl2 (SEQ ID NO: 10, the sequence linking the Hes5 promoter and Plagl2 is SEQ ID NO: 11) most efficiently increased the number of MCM2⁺ mitotic cells (activated NSCs, cells likely to be intermediate progenitor cells) in the hippocampal dentate gyrus of the 6-month-old mice as compared with the control mouse.

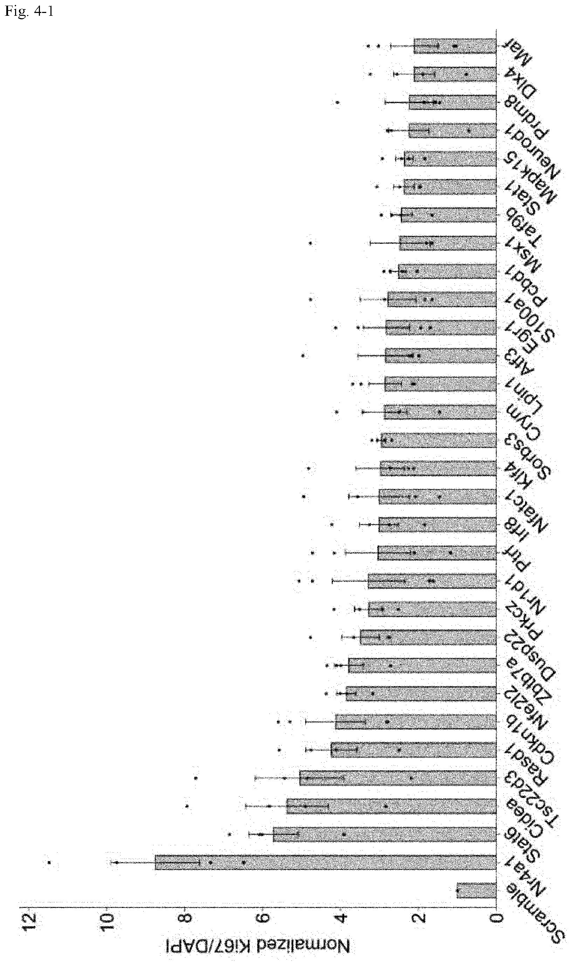

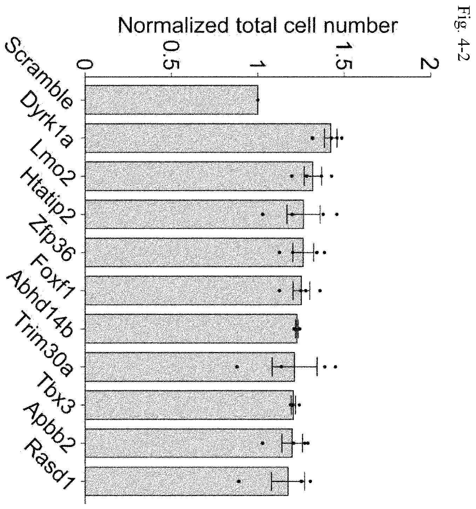

Next, in order to identify genes that can proliferate and activate NSCs by suppressing the expression thereof, genes that are low expressed in an embryo and highly expressed in adult NSCs were used as subject genes. siRNAs of targeted subject genes were introduced into quiescent NSCs to carry out knockdown of genes expressed at a high level in adult NSCs ("adult-high") and genes of modulators therefor. Results of in vitro knockdown screening are shown in Figure 4-1 and Figure 4-2. Among the 124 genes tested, 11 genes whose knockdown efficiently activated quiescent NSCs were selected.

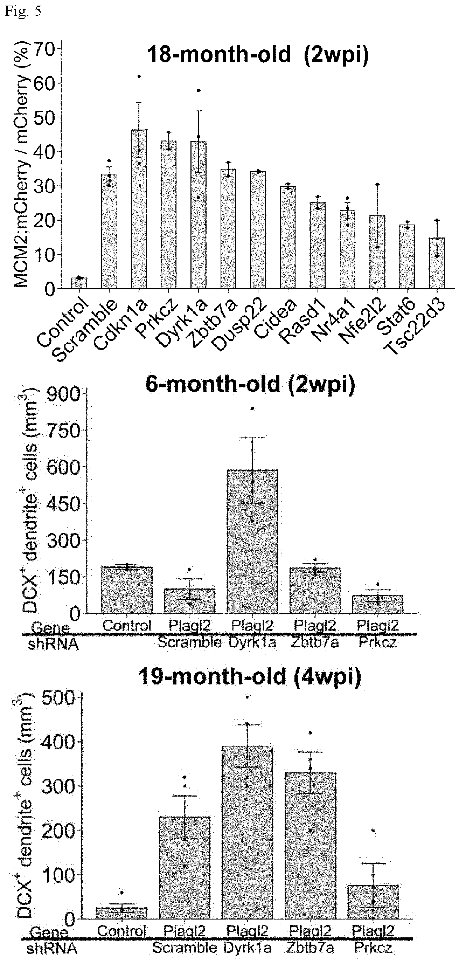

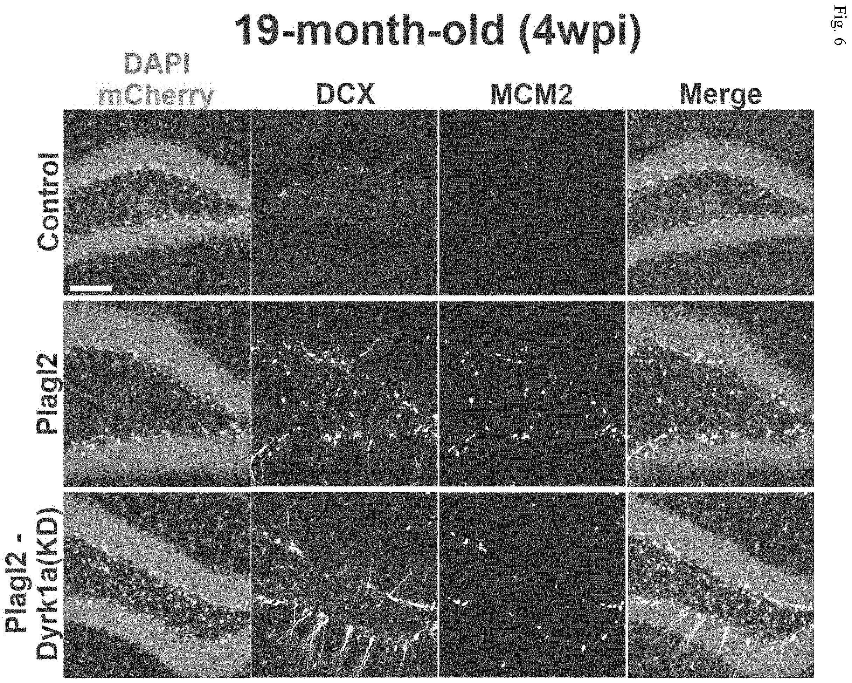

By using a lentivirus, expression of an shRNA of each of the 11 genes selected above was induced in vivo with Plagl2 under the control of the Hes5 promoter (SEQ ID NO: 9). Each lentivirus was injected into the hippocampal dentate gyrus of 6-, 18-, or 20-month-old mice, and brain sections were investigated after 2 or 4 weeks. As a result, shRNAs of Cdkn1a, Prkcz, and Dyrk1a increased the number of MCM2⁺ active NSCs/IPCs in the 18-month-old mice (top in Fig. 5). "Control" in Fig. 5 shows the results obtained by connecting only mCherry to the Hes5 promoter, and "Scramble" shows the results obtained by connecting mCherry, Plagl2, and a random sequence to the Hes5 promoter. Determination of an increase or a decrease in the number of MCM2⁺ active NSCs/IPCs is based on comparison with "Scramble." In addition, shRNA (SEQ ID NO: 13) of Dyrk1a (SEQ ID NO: 12) more efficiently increased the number of DCX⁺ dendrite⁺ cells than others in 6-month-old and 19-month-old (18 months + 4 weeks) mice (center in Fig. 5, bottom in Fig. 5, Fig. 6). The center in Fig. 5 and the bottom in Fig. 5 show the number of DCX⁺ dendrite⁺ cells in 6-month-old and 19-month-old (18 months + 4 weeks) mice in which expression of shRNAs of Prkcz, Dyrk1a, and Zbtb7a, where the number of MCM2⁺ active NSCs/IPCs tended to increase, was induced. The above IPCs are intermediate progenitor cells.

These data show that combined use of Plagl2 overexpression and Dyrk1a knockdown most efficiently activate neurogenesis in the hippocampal dentate gyrus of both 6-month-old and 19-month-old mice, and the combined use of the two gene expressions was termed iPaD (inducing Plagl2 and anti-Dyrk1a activity). iPaD (SEQ ID NO: 14), which is this combination of the two gene expressions, increased the number of MCM2⁺ and/or BrdU-incorporating cells, including Sox2⁺ GFAP⁺ BrdU⁺ activated NSCs and DCX⁺ dendrite⁺ BrdU⁺ immature neurons. These results suggest the possibility that Plagl2 overexpression and Dyrk1a knockdown in endogenous neural stem cells in an adult brain, an aged brain, or the like efficiently activates and proliferates these neural stem cells to produce a large number of neurons, resulting in improved memory and learning ability.

### 2. Long-term effects of iPaD on neurogenesis

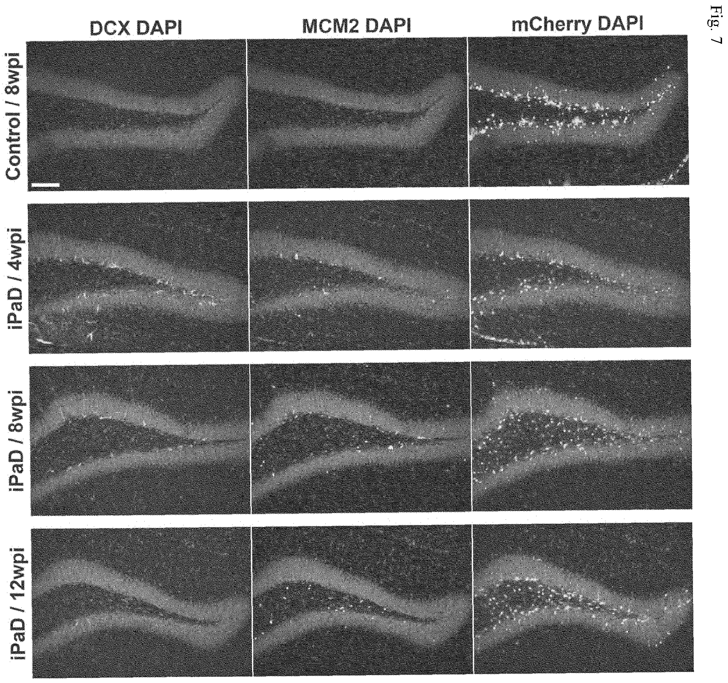

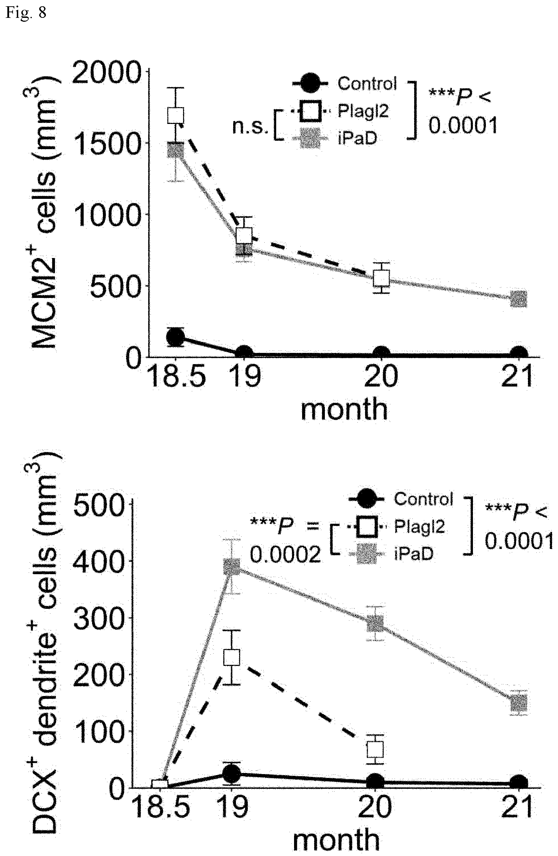

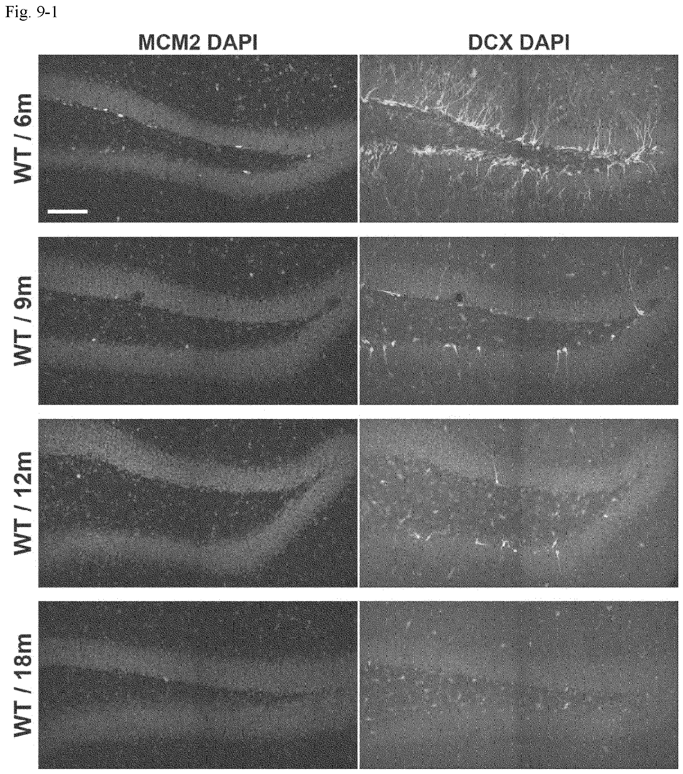

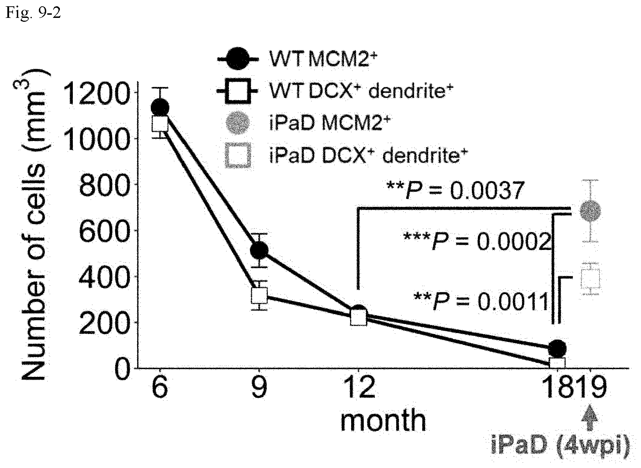

In order to investigate the long-term effects of an iPaD lentivirus on neurogenesis in an aged brain, the iPaD lentivirus was introduced into the hippocampal dentate gyrus of 18-month-old mice. Only a few activated NSCs (MCM2⁺;DCX⁻), IPCs (MCM2⁺;DCX^{+/-}), and immature neurons (MCM2⁻;DCX⁺;dendrite⁺) were present in this region in the mice at this age in months, and the control lentivirus did not affect the numbers thereof (Control/8wpi in Fig. 7). On the other hand, the iPaD lentivirus efficiently activated NSCs and induced the formation of IPCs and immature neurons 1 month after infection (19 months of age) (iPaD/4wpi in Fig. 7). A similar effect was observed even at week 8 and week 12 after infection (20 months of age and 21 months of age, respectively) (iPaD/8wpi and iPaD/12wpi, respectively, in Fig. 7), and thus it was suggested that neurogenesis is continuously activated by iPaD lentivirus-induced Plagl2 overexpression and Dyrk1a knockdown in endogenous neural stem cells in the aged brain. Plagl2 alone also activated neurogenesis, but less efficiently than the iPaD lentivirus (Fig. 8). Combining Plagl2 overexpression with Dyrk1a knockdown resulted in remarkable effects on activation of neural stem cells and the formation of neurons. These results suggested that NSCs activated by the iPaD lentivirus proliferate continuously and are not exhausted even after 3 months. In wild-type hippocampi, MCM2⁺ or DCX⁺ cells decreased significantly in number with aging, and almost disappeared at 18 months of age (Fig. 9-1). However, the iPaD lentivirus increased the number of these cells in the hippocampal dentate gyrus of the aged brain (19 months of age) to a level comparable to that in 9-month-old or even younger wild-type mice (Fig. 9-2), and thus it was suggested that neurogenesis in the aged brain can be effectively reactivated by the iPaD lentivirus.

In order to check whether or not the iPaD lentivirus has a therapeutic effect on Alzheimer's disease, the iPaD lentivirus was introduced into the hippocampal dentate gyrus of Alzheimer model mice, and the same analysis as above was carried out. As a result, the iPaD lentivirus efficiently activated NSCs in the Alzheimer model mice and induced the formation of IPCs and immature neurons. In addition, amyloid β deposition was suppressed, and memory improvement was observed in fear conditioning experiments. The details thereof are described in the following section "4. Therapeutic effects of iPaD on Alzheimer's disease."

In addition, in order to investigate whether or not the iPaD lentivirus can activate non-NSCs, the iPaD lentivirus was injected into a brain region in which no neural stem cells were present. The Hes5 promoter is also active in astrocytes, but none expressed MCM2 or DCX, and thus it is suggested that the activation induced by the iPaD lentivirus is specific to NSCs.

### 3. Improvement of cognitive functions by iPaD (Barnes maze test)

In order to investigate whether or not activated neurogenesis improves cognitive functions in aged mice, spatial learning and memory were analyzed with a Barnes maze test. By using this test, it was previously shown that spatial learning and memory depend on neurogenesis in the hippocampal dentate gyrus (Imayoshi, I., et al. Roles of continuous neurogenesis in the structural and functional integrity of the adult forebrain. Nat Neurosci 11, 1153-1161 (2008)), and it was shown that aged mice require longer distances and latencies to reach the target hole in the training session and exhibit poorer performance than young mice (Shoji, H. & Miyakawa, T. Age-related behavioral changes from young to old age in male mice of a C57BL/6J strain maintained under a genetic stability program. Neuropsychopharmacol. Rep. 39, 100-118 (2019)).

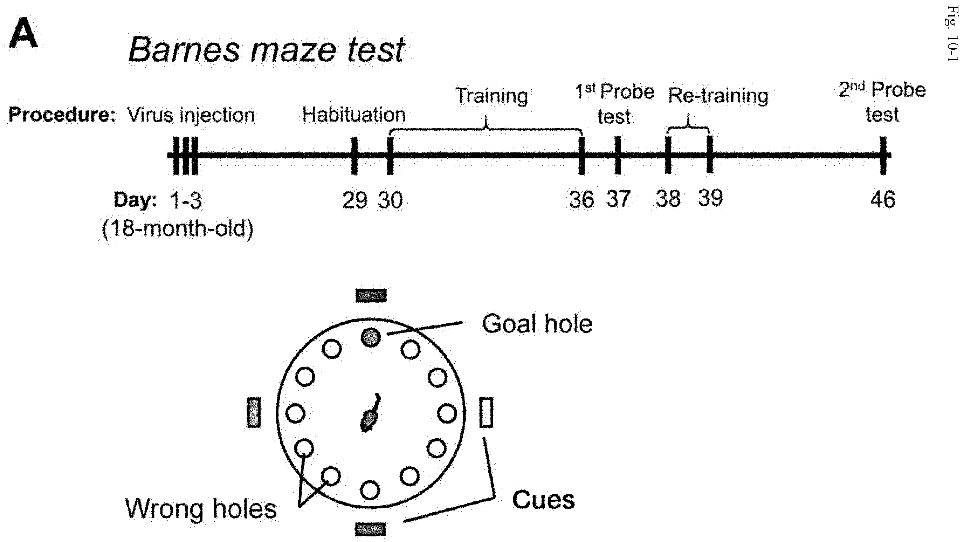

19-month-old mice were divided into two groups, which were then injected with the control lentivirus and the iPaD lentivirus, respectively, before one month (Fig. 10-1). The Barnes maze test was carried out on these mice as follows. That is, 10 to 12 male mice per group were used for each behavior analysis. Automatic mouse tracking was carried out by using ANY-maze software (Stoellting Co.) and detecting a plurality of body points. One day before virus injection, 10 mg/kg of P7C3-A20 (MedKoo) was administered by intraperitoneal injection once daily until sacrifice in order to reverse surgical damage. P7C3-A20 was dissolved in a solution of 5% dextrose, 3% DMSO, and 10% Cremophor EL (Nacalai). Spatial memory was measured by using a Barnes circular maze (92 cm in diameter, 12 holes, Brain Science Idea Co., Ltd.). The behavior test space was surrounded by a black curtain, and 4 representative cues were placed at least 30 cm away from the edge of the maze. Habituation was carried out with one escape hole for 5 minutes. One day after a habituation phase, each mouse was trained twice daily to memorize the location of one escape hole, which was randomized among mice but fixed for each individual mouse. At the beginning of the training, a mouse was placed in a white cylinder, which was located in the center of the maze for 30 seconds, and the cylinder was configured to be uncovered when recording started. Each recording was automatically stopped up to 5 minutes after the mouse entered the escape hole or hovered around the escape hole for 10 seconds. When the mouse did not find the escape hole during the 5-minute training, the experimenter gently guided the mouse to the escape hole. The mouse was left undisturbed in the escape hole for 60 seconds after entering the escape hole. After training, the first probe test was carried out for 3 minutes at intervals of one day. During the training, a camera placed directly above the maze recorded the total distance, the number of times the mouse circled around the correct hole and wrong holes, and the latency to enter the goal hole. In the probe test, the total distance, the number of visits, and the time spent around the correct and wrong holes were recorded. The intensity of light was set to 100 lux on training day 1, and increased by about 30 lux per day. The maze was thoroughly washed with a 70% ethanol solution and dried after every trial.

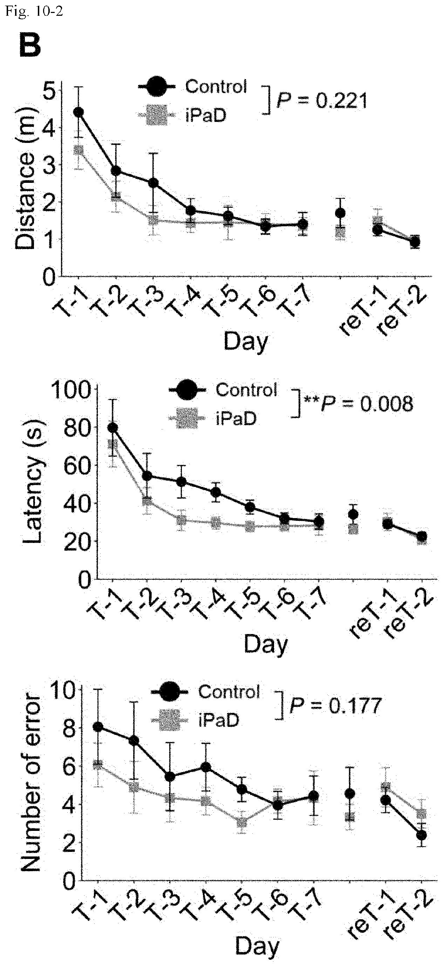

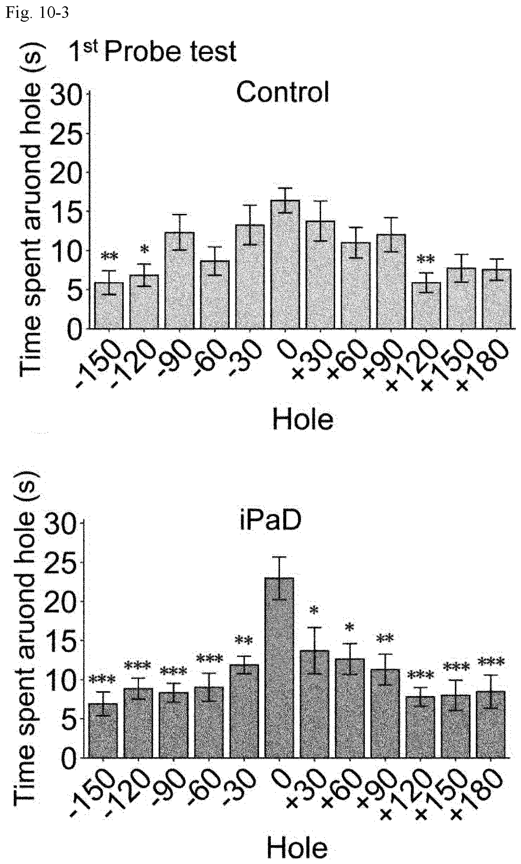

As a result, it was found that mice injected with the iPaD lentivirus reached the target hole at shorter distances, made fewer errors, and had shorter latencies than mice injected with the control lentivirus (Fig. 10-2). In the first probe test (1 day after training), iPaD mice stayed longer in the target hole than control mice (Fig. 10-3). These data show that injection of the iPaD lentivirus improved the cognitive functions of aged mice in the Barnes maze test.

### 4. Comparison between Alzheimer's disease model mice and normal mice

### (1) Comparison of the number of MCM2-positive cells and the number of DCX-positive cells in hippocampal dentate gyrus and amyloid β deposition

5 × FAD mice were used as Alzheimer's disease model mice. The 5 × FAD mice are (Tg6799 line) mice into which a mutant human amyloid precursor protein (APP) (Swedish mutation: K670N, M671L; Florida mutation: I716V; London mutation: V717I) gene and a mutant human presenilin 1 (PS 1) (M146L; L286V) gene have been introduced under the control of the mouse Thy1 promoter. In these mice, amyloid β is deposited in a brain region, neurogenesis is also reduced, and memory and learning abilities are impaired. The 5 × FAD mice (B6/SJL genetic background) were purchased from Jackson Laboratory and crossed with C57BL/6J wild-type mice for 2 generations or more before use. Wild-type littermate mice were used as control animals. All mice were handled according to the "Regulations on Animal Experimentation at Kyoto University." The experimental protocol was approved by the Animal Experimentation Committee of the Institute for Frontier Life and Medical Sciences, Kyoto University.

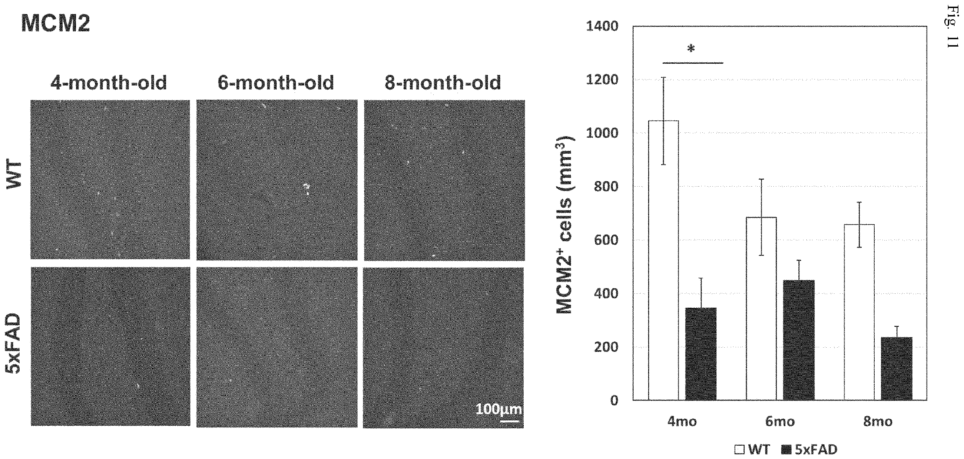

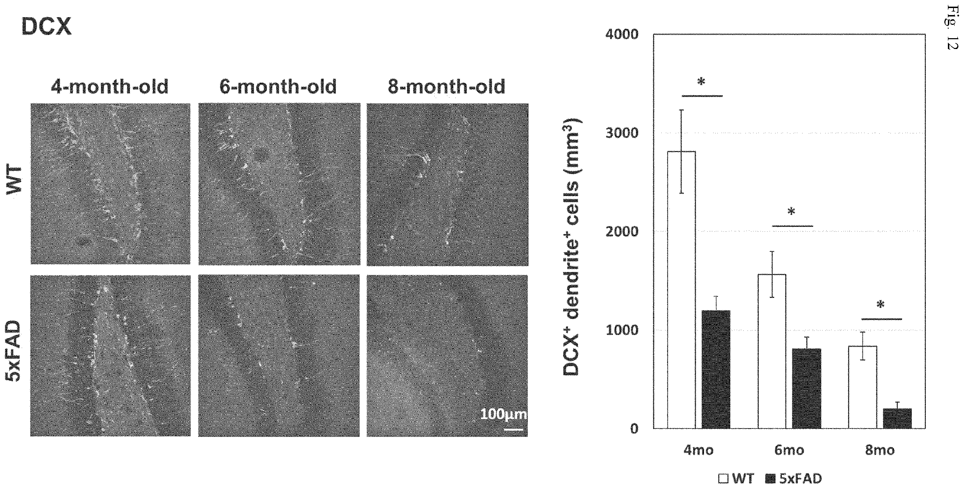

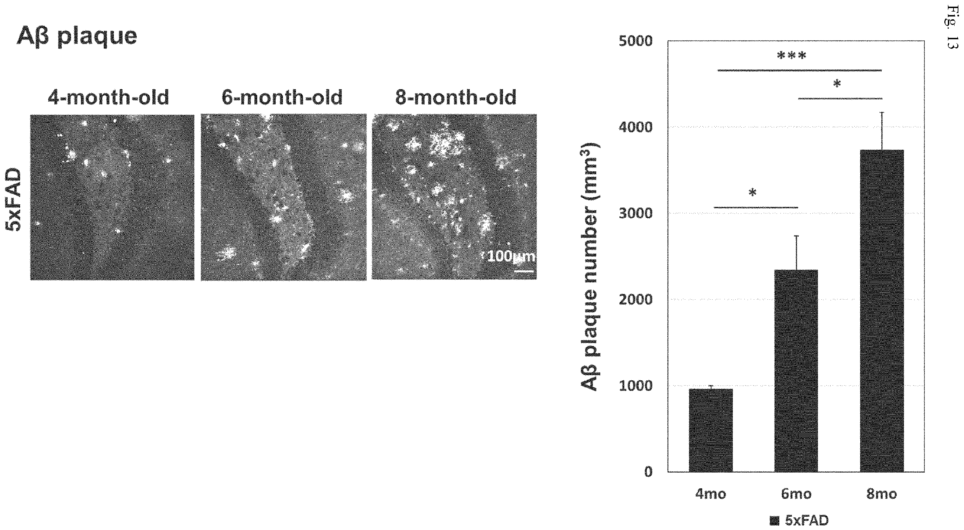

Sections of the hippocampal dentate gyrus were prepared from 4-month-old, 6-month-old, and 8-month-old Alzheimer's disease model mice (5 × FAD mice) and normal mice (wild-type littermate mice), and immunostained to determine the number of MCM2-positive cells and the number of DCX-positive cells to check changes with age in months. Results thereof are shown in Fig. 11 (MCM2-positive cells) and Fig. 12 (DCX-positive cells). Quantitative evaluation of labeled cells was carried out according to the method described in the section "Quantitative evaluation of labeled cells." In addition, sections of the hippocampal dentate gyrus were prepared from 4-month-old, 6-month-old, and 8-month-old Alzheimer's disease model mice (5 × FAD mice), and changes with age in months in amyloid β deposition were also checked. Results thereof are shown in Fig. 13. Quantitative evaluation of amyloid β deposition was carried out according to the method described in the section "Quantitative evaluation of amyloid β deposition."

### (Quantitative evaluation of labeled cells)

The hippocampal dentate gyrus of 3 or more mice in each experiment was used for analysis. For each animal, 50 µm thick serial sections of the hippocampal dentate gyrus anterior to the iPaD lentivirus injection point were prepared and evaluated every seventh section. All sections except damaged sections were used for immunostaining. After immunostaining, z-stack images were acquired by using a confocal microscope (LSM780) with a 10× or 20× objective lens. Stained cells were counted by using Imaris software (Bitplane) or ImageJ and quantified as the number of cells per mm³ of the hippocampal dentate gyrus.

### (Quantitative evaluation of amyloid β deposition)

Every seventh brain section from each mouse was labeled with a mouse anti-β-amyloid primary antibody (Clone 6E10; 1:500; BioLegend 9320) and detected with a fluorescently labeled secondary antibody. After immunostaining, z-stack images were acquired by using a confocal microscope (LSM780) with a 10× objective lens. The number of Aβ plaques was quantified by using ImageJ after adequate thresholding and denoising. Results thereof were expressed as the number of plaques per mm³ of the hippocampal dentate gyrus.

As shown in Fig. 11, the number of cells positive for MCM2, a marker for activated NSCs, was about 1000 cells/mm³ in normal mice at 4 months of age, and decreased with increasing months of age, reaching 700 cells/mm³ at 6 months of age, and about 680 cells/mm³ at 8 months of age. On the other hand, in the Alzheimer's disease model mice (5 × FAD mice), the number of MCM2-positive cells was as small as about 350 cells/mm³ even at 4 months of age, and remained small with no great change even with increasing months of age.

As shown in Fig. 12, the number of cells positive for DCX, an undifferentiated neuron marker, was about 2700 cells/mm³ in normal mice at 4 months of age, and decreased with increasing months of age, reaching 1500 cells/mm³ at 6 months of age, and about 800 cells/mm³ at 8 months of age. On the other hand, in the Alzheimer's disease model mice (5 × FAD mice), the number of DCX-positive cells was as small as about 1200 cells/mm³ even at 4 months of age, and further decreased with increasing months of age, reaching about 800 cells/mm³ at 6 months of age and about 150 cells/mm³ at 8 months of age.

As shown in Fig. 13, in the Alzheimer's disease model mice (5 × FAD mice), amyloid β deposition (amyloid β plaques) remarkably increased with increasing months of age, and was about 1000 plaques/mm³ at 4 months of age, reaching about 2400 plaques/mm³ at 6 months of age, and about 3700 plaques/mm³ at 8 months of age.

### (2) Behavior tests

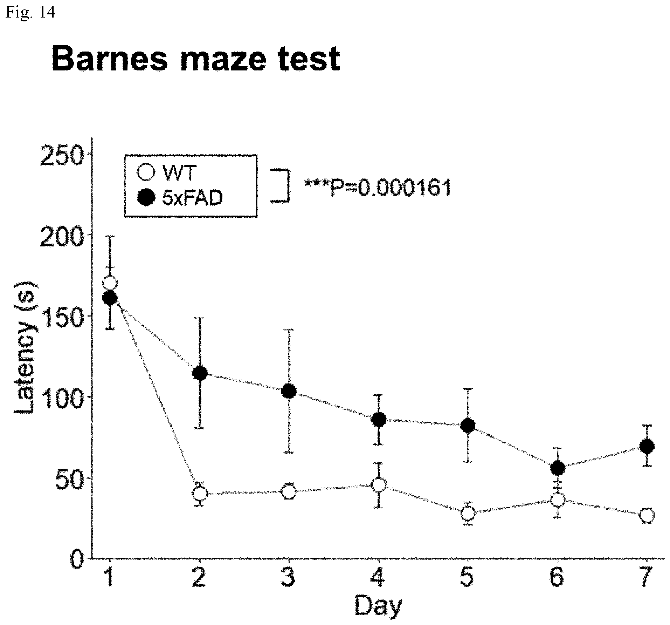

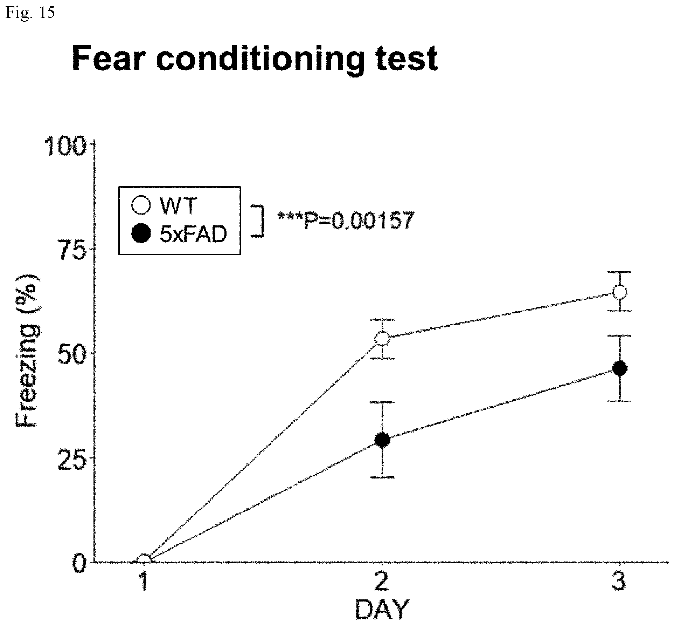

Behavior tests (Barnes circular maze test, contextual fear conditioning test) were carried out on Alzheimer's disease model mice (5 × FAD mice) and normal mice (wild-type littermate mice) according to the methods described below. Results thereof are shown in Fig. 14 (Barnes circular maze test) and Fig. 15 (contextual fear conditioning test).

8 to 12 male mice per group were used for each behavior analysis. Automatic mouse tracking was carried out by using ANY-maze software (Stoelting Co.) to detect a plurality of body points. In the case of a test with virus injection, 10 mg/kg of P7C3-A20 (manufactured by MedKoo) was administered intraperitoneally once daily for one week from 1 day before virus injection to reverse surgical damage. P7C3-A20 was dissolved in a solution of 5% dextrose, 3% DMSO, and 10% Cremophor EL (Nacalai).

Spatial memory was checked by the same Barnes maze test as in the above section "3. Improvement of cognitive functions by iPaD (Barnes maze test)."

Contextual memory was evaluated by a contextual fear conditioning test with partial modification of the protocol described in a previous report (Walgrave, H. et al., Cell Stem Cell 28, 1805-1821.e1808, doi:10.1016/j.stem.2021.05.001 (2021)). Twenty-four stainless steel rods wired to an electric shock generator were attached to the floor of a chamber (17 × 10 × 10 cm), and the test was carried out in an acoustically attenuated cubicle having 60 dB of background white noise. On day 1, each mouse was placed in a chamber (context A, floor: stainless steel rods, walls: colorless and transparent) and allowed to explore for 3 minutes. After that, a foot shock of 80 mA for 2 seconds was applied. The mouse was allowed to remain in the chamber for an additional minute. The chamber was disinfected with 70% ethanol and dried for each animal. After 24 and 48 hours, the mouse was placed back in context A, and the same procedure as on day 1 was repeated. By using TimeFZ2 (O'HARA & CO., LTD, Japan), fear responses during the first 3 minutes were measured.

In the present Example, the data are expressed as mean ± standard error (SEM). Statistical analysis was carried out by using R software. Statistical differences were examined by using one-way analysis of variance or two-way analysis of variance, and further, a Tukey-Kramer method or a Bonferroni method was used as a post hoc test. A P-value < 0.05 was considered significant.

As shown in Figs. 14 and 15, it can be seen that the Alzheimer's disease model mice (5 × FAD mice) are inferior to the normal mice in both spatial memory ability and contextual memory ability.

### 5. Therapeutic effects of iPaD on Alzheimer's disease

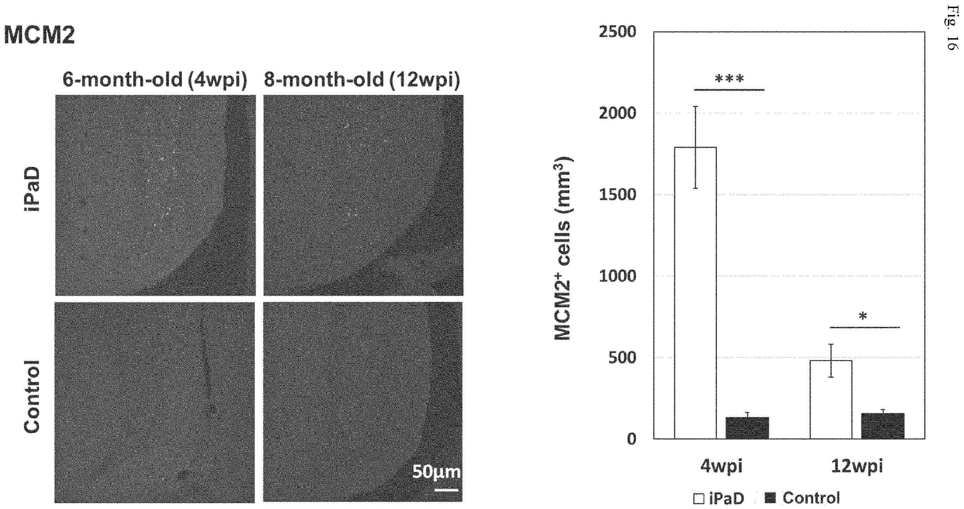

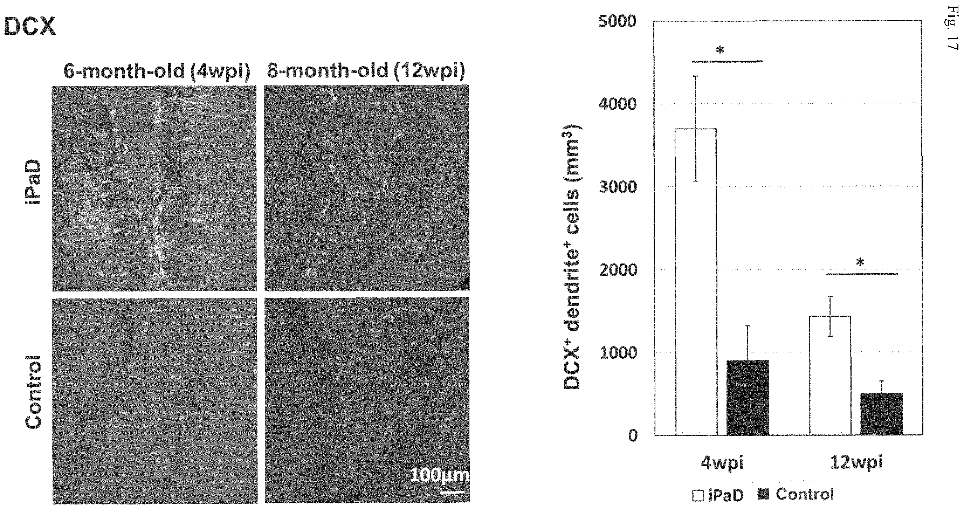

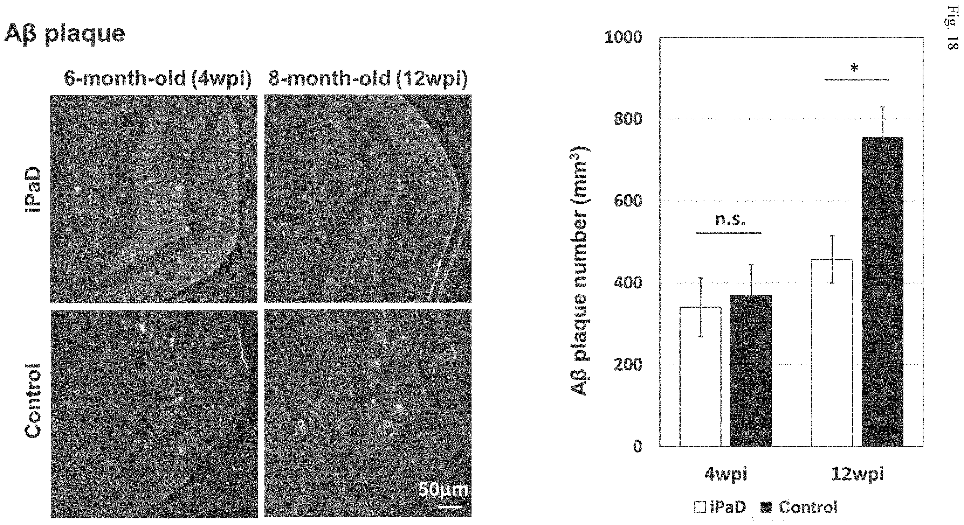

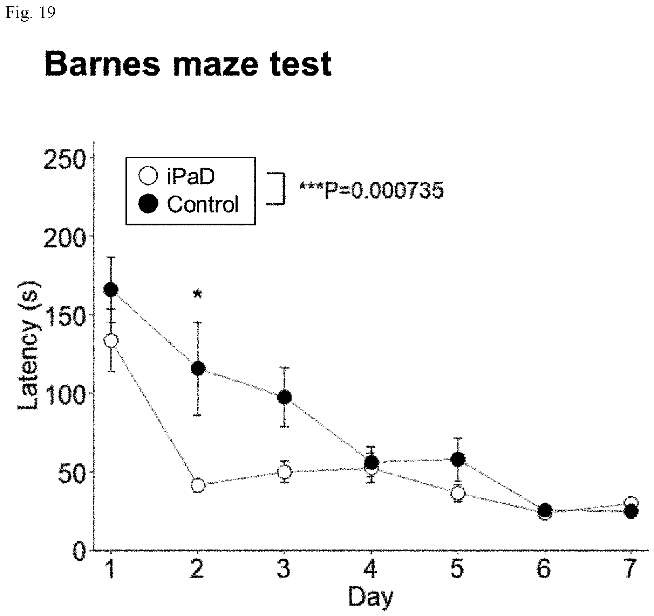

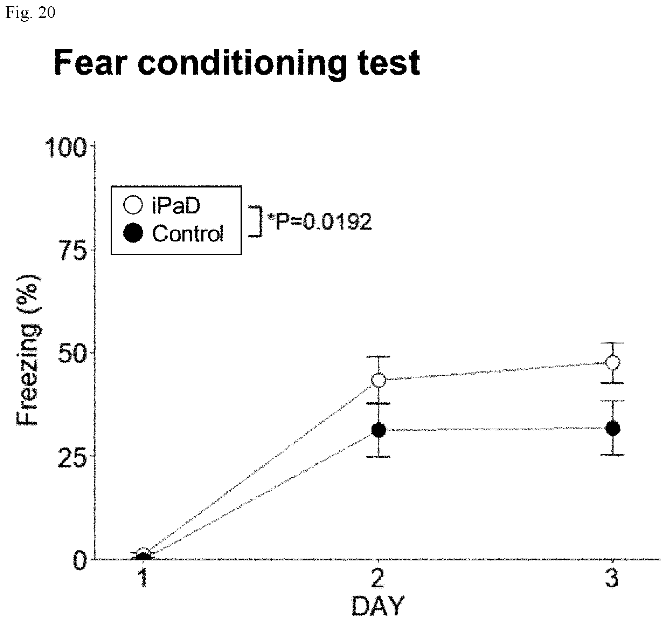

In order to investigate whether or not the iPaD lentivirus has a therapeutic effect on Alzheimer's disease, the control lentivirus or the iPaD lentivirus was introduced into the hippocampal dentate gyrus of 5-month-old 5 × FAD mice. After 4 weeks (6 months of age) and after 12 weeks (8 months of age), 50 µm thick serial sections of the hippocampal dentate gyrus anterior to the lentivirus injection point were prepared, and the number of MCM2-positive cells, the number of DCX-positive cells, and amyloid β deposition was checked and evaluated every seventh section. In addition, behavior tests (Barnes circular maze test and contextual fear conditioning test) were also carried out 4 weeks after the introduction of the iPaD lentivirus. Results thereof are shown in Fig. 16 (MCM2-positive cells), Fig. 17 (DCX-positive cells), Fig. 18 (amyloid β deposition), Fig. 19 (Barnes circular maze test), and Fig. 20 (contextual fear conditioning test). The quantitative evaluation of labeled cells was carried out according to the method described in the above section "Quantitative evaluation of labeled cells," and the quantitative evaluation of amyloid β deposition was carried out according to the method described in the above section "Quantitative evaluation of amyloid β deposition,", the Barnes circular maze test was carried out according to the method described in the above section "3. Improvement of cognitive functions by iPaD (Barnes maze test)," the contextual fear conditioning test was carried out according to the method described in "(2) Behavior tests" in the above section "4. Comparison between Alzheimer's disease model mice and normal mice."

As shown in Fig. 16, in the iPaD lentivirus-introduced mice, the number of cells positive for MCM2, a marker for active NSCs, remarkably increased at week 4 after administration. At week 12 after administration, the number decreased from that at week 4, but was significantly larger than in the control.

As shown in Fig. 17, in the iPaD lentivirus-introduced mice, the number of cells positive for DCX, an immature neuron marker, remarkably increased at week 4 after administration. At week 12 after administration, the number decreased from that at week 4, but was significantly larger than in the control.

As shown in Fig. 18, in amyloid β deposition, at week 4 after the introduction of the iPaD lentivirus, there was no difference from the control lentivirus-introduced control, but at week 12 after introduction, a remarkable suppression effect was observed. That is, in the control, the number of amyloid β plaques increased to twice or more that at week 4, whereas in the iPaD lentivirus-introduced mice, the increase was suppressed to about 1.2 times that at week 4.

As shown in Fig. 19 and Fig. 20, in the iPaD lentivirus-introduced mice, spatial memory and contextual memory were restored to the levels thereof in the normal mice shown in Fig. 14 and Fig. 15, respectively.

As described above, it was found that the introduction of the iPaD lentivirus remarkably improved a symptom of Alzheimer's disease and provided excellent therapeutic effects.

### 6. Induction of differentiation from inner ear supporting cells to hair cells by iPaD

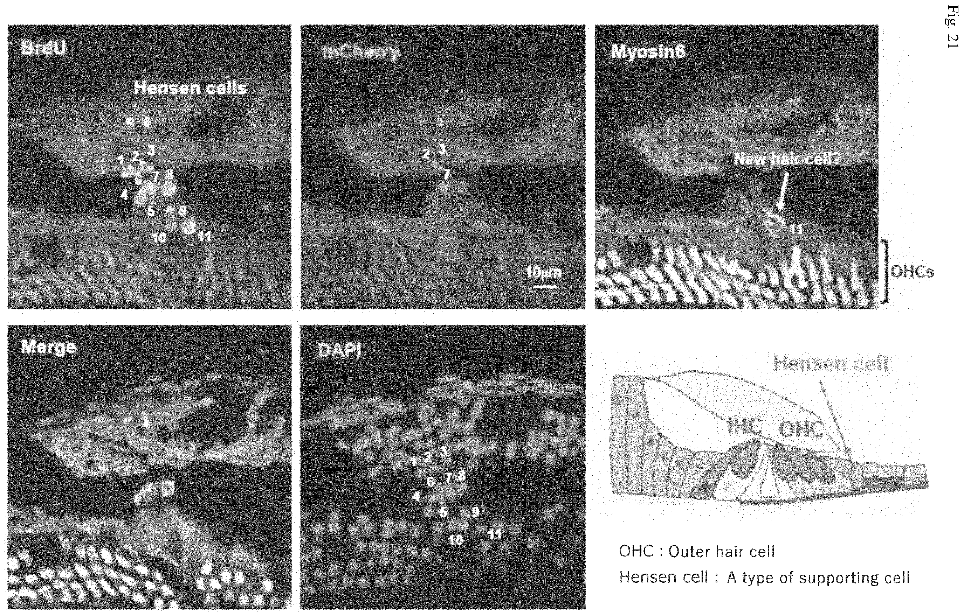

iPaD was expressed in supporting cells (Hensen cells) of the inner ear of one-month-old young mice for two weeks. In many cases, a population consisting of several BrdU⁺ cells was formed (one supporting cell is thought to have divided into several cells), but the population shown in this figure consisted of 11 BrdU+ cells (Fig. 21). It is considered that one supporting cell divided into 11 cells. Among these, 3 cells were mCherry+ (Nos. 2, 3, 7). In the inner ear, the Hes5 promoter works weakly only in supporting cells but does not work in other cells, and thus it was suggested the possibility that eight mCherry-negative cells changed from supporting cells to other cell types. One (No. 11) of these cells was Myosin6-positive and was considered to be differentiated into a hair cell. Supporting cells at this stage have already lost the cell proliferation ability thereof, and thus it was suggested the possibility that iPaD can efficiently restore the proliferation ability of the cells and differentiate at least some thereof into hair cells.

### INDUSTRIAL APPLICABILITY

According to the present invention, by introducing a specific gene set into an adult brain or an aged brain, endogenous neural stem cells can be efficiently activated and proliferated to produce a large number of neurons. The introduction thereof can cause neural stem cells in the aged brain to revert to their adolescent or younger state and can produce a large number of neurons, resulting in improved memory and learning abilities. In addition, a therapeutic effect thereof on a neurodegenerative disease such as Alzheimer's disease can also be expected, and endogenous neural stem cells can be efficiently activated to produce a large number of neurons, resulting in improved memory and learning abilities. Further, the specific gene set of the present disclosure can differentiate not only brain neural stem cells but also supporting cells in the inner ear into hair cells, and it is suggested that the specific gene set is also effective in the treatment of an inner ear disease.

## Claims

1. A polynucleotide comprising:
(A) a nucleic acid sequence of a Plagl2 gene;
(B) a nucleic acid sequence of an miR-shRNA (microRNA adapted short hairpin RNA) against a Dyrk1a gene; and
(C) a promoter sequence operably linked to the above nucleic acid sequences.

2. The polynucleotide according to claim 1, wherein the Plagl2 gene and the Dyrk1a gene are mammalian genes.

3. The polynucleotide according to claim 1 or 2, wherein the promoter is selected from the group consisting of a Hes5 promoter, a GFAP promoter, a Sox2 promoter, and an Lfng promoter.

4. A vector comprising the polynucleotide according to any one of claims 1 to 3.

5. The vector according to claim 4, wherein the vector is a lentivirus vector, an adeno-associated virus vector, an adenovirus vector, or a plasmid vector.

6. A pharmaceutical composition comprising the vector according to claim 4 or 5.

7. A pharmaceutical composition comprising:
(a) a vector comprising a nucleic acid sequence of a Plagl2 gene and a promoter sequence operably linked to the nucleic acid sequence of the Plagl2 gene; and
(b) a vector comprising a nucleic acid sequence of an miR-shRNA (microRNA adapted short hairpin RNA) against a Dyrk1a gene and a promoter sequence operably linked to the nucleic acid sequence of the miR-shRNA, or an siRNA or an miRNA against the Dyrk1a gene.

8. Cells transformed with the vector according to claim 4 or 5.

9. A pharmaceutical composition comprising the cells according to claim 8.

10. The pharmaceutical composition according to claim 6, 7, or 9, wherein the pharmaceutical composition is used for treatment of a neurodegenerative disease or an inner ear disease.

11. The pharmaceutical composition according to claim 10, wherein the pharmaceutical composition is administered to a subject by intracerebroventricular injection, intrathecal bolus injection or infusion, intraganglionic injection, intraneural injection, subcutaneous injection, or intratympanic injection.

12. A method for screening for a substance for treatment of a neurodegenerative disease or an inner ear disease, comprising:
(1) a step of causing a test substance to act on neural stem cells or inner ear supporting cells to measure expression of a Plagl2 gene and a Dyrk1a gene; and
(2-1) a step of selecting a test substance having an ability to enhance Plagl2 gene expression and an ability to suppress Dyrk1a gene expression from a result of the step (1) or
(2-2) a step of selecting a test substance having an ability to enhance Plagl2 gene expression, and a test substance having an ability to suppress Dyrk1a gene expression from the result of the step (1).
